# EUROPÄISCHE PATENTSCHRIFT

(11) **EP 1 868 600 B1**
(45) Veröffentlichungstag und Bekanntmachung des Hinweises auf die Patenterteilung: **27.06.2012**
(21) Anmeldenummer: 06724117.4
(22) Anmeldetag: 07.04.2006
(51) Int. Cl.: A61K 31/407, A61K 31/438, A61K 31/485, A61K 45/06, A61P 25/36

(54) **SPIROCYCLISHE CYCLOHEXANDERIVATE ZUR BEHANDLUNG VON SUBSTANZABHÄNGIGKEIT**
SPIROCYCLIC CYCLOHEXANE DERIVATIVES FOR USE IN THE TREATMENT OF SUBSTANCE DEPENDENCY
DERIVES DE CYCLOHEXANE SPIROCYCLIQUES PERMETTANT DE TRAITER UNE DEPENDANCE A UNE SUBSTANCE

(30) Priorität: 11.04.2005 DE 102005016460
(43) Veröffentlichungstag der Anmeldung: 26.12.2007
(73) Patentinhaber: Grünenthal GmbH, 52078 Aachen (DE)
(72) Erfinder: FRIDERICHS, Elmar, 52223 Stolberg (DE); KÖGEL, Babette-Yvonne, 52379 Langerwehe (DE); LINZ, Klaus, 53343 Wachtberg (DE)
(86) Internationale Anmeldenummer: PCT/EP2006/003176
(87) Internationale Veröffentlichungsnummer: WO 2006/108565

(56) Entgegenhaltungen:
- WO-A-03/039469
- WO-A-2004/043967
- WO-A-2005/066183
- ZAVERI NURULAIN: "Peptide and nonpeptide ligands for the nociceptin/orphanin FQ receptor ORL1: Research tools and potential therapeutic agents." LIFE SCIENCES, Bd. 73, Nr. 6, 27. Juni 2003 (2003-06-27), Seiten 663-678, XP002393013 ISSN: 0024-3205
- CALO' G ET AL: "Pharmacology of nociceptin and its receptor: a novel therapeutic target" BRITISH JOURNAL OF PHARMACOLOGY, BASINGSTOKE, HANTS, GB, Bd. 129, Nr. 7, April 2000 (2000-04), Seiten 1261-1283, XP002303597 ISSN: 0007-1188
- KOTLINSKA JOLANTA ET AL: "Orphanin FQ/nociceptin inhibits morphine withdrawal" LIFE SCIENCES, Bd. 66, Nr. 8, 14. Januar 2000 (2000-01-14), Seiten 119-123, XP002393014 ISSN: 0024-3205
- DI GIANNUARIO AMALIA ET AL: "Orphanin FQ reduces morphine-induced dopamine release in the nucleus accumbens: A microdialysis study in rats" NEUROSCIENCE LETTERS, Bd. 272, Nr. 3, 17. September 1999 (1999-09-17), Seiten 183-186, XP002393015 ISSN: 0304-3940
- MURPHY NIALL P ET AL: "Orphanin FQ/nociceptin blocks acquisition of morphine place preference" BRAIN RESEARCH, Bd. 832, Nr. 1-2, 19. Juni 1999 (1999-06-19), Seiten 168-170, XP002393016 ISSN: 0006-8993
- BIGNAN G C ET AL: "Recent advances towards the discovery of ORL-1 receptor agonists and antagonists" EXPERT OPINION ON THERAPEUTIC PATENTS 2005 UNITED KINGDOM, Bd. 15, Nr. 4, 2005, Seiten 357-388, XP002393017 ISSN: 1354-3776
- SHOBLOCK JAMES R ET AL: "The effect of a systemically active ORL-1 agonist, Ro 64-6198, on the acquisition, expression, extinction, and reinstatement of morphine conditioned place preference" NEUROPHARMACOLOGY, Bd. 49, Nr. 4, September 2005 (2005-09), Seiten 439-446, XP002393018 ISSN: 0028-3908

## Beschreibung

Die Opiatabhängigkeit stellt ein großes medizinisches und soziales Problem dar, das einer intensiven medizinischen Betreuung bedarf. Als Standardtherapie gilt die Substitution mit einem speziell geeigneten µ-Opioid.

Klinisch etablierte Substanzen für die Substitutionstherapie der Opiatabhängigkeit sind:
- Methadon (Razemat, seltener Levomethadon)
- LAAM (α-Acetylmethadol)
- Buprenorphin

Alle drei Substanzen werden mit der gleichen Zielsetzung angewendet:
- Befriedigung des Opiat"hungers" jedoch ohne "Kick" und bei deutlich verringerter Euphorie
- Vermeidung von körperlichen Entzugssymptomen
- Resozialisierung der Patienten (geregelte Lebensführung, Arbeitsfähigkeit, Ausstieg aus dem Fixermilieu)
- Langfristige Reduktion der Opiatdosis bis zur vollständigen Abstinenz

Damit diese Wirkungen erreicht werden können sind folgende Eigenschaften bei den Substitutions-Therapeutika erforderlich:
- Orale (ev. auch cutane) Wirkverfügbarkeit
- Lange Wirkdauer (1 - 3 Tage, ev. auch länger)
- Hohe Wirkstärke (Potency)
- Geringe Euphorisierung
- Wenig "Kick"
- Geringe Entzugssymptomatik
- Gute Langzeitverträglichkeit (wenig Obstipation, cardiovasculäre Verträglichkeit)

Diese Eigenschaften sind bei den bisherigen Standardtherapeutika nur teilweise gegeben, so dass es zu Unzulänglichkeiten beim therapeutischen Einsatz kommt.

Die wesentlichen Probleme liegen bei den derzeitigen Standardtherapeutika im folgenden: Methadon besitzt ein hohes Missbrauchspotential. Aufgrund der mäßigen Wirkdauer ist eine tägliche Gabe erforderlich. Darüber hinaus tritt als Nebenwirkung Obstipation auf.
LAAM besitzt den Nachteil einer hohen Kardiotoxizität und kann Torsade de Pointes-Arrhythmien auslösen.
Buprenorphin weist eine hohe Toxizität in Kombination mit Benzodiazepinen auf.

In WO 0443967 und PCT/EP2004/014539 werden Verbindungen beschrieben, die insbesondere zur Behandlung von Schmerz, aber auch unter anderem zur Behandlung von Entzugserscheinungen sowie zur Reduzierung des Suchtpotentials von Opioiden geeignet sind. Es konnte gezeigt werden, dass ausgewählte Verbindungen aus dieser Patentanmeldung in besonderer Weise als Substitutionstherapeutika geeignet sind.

Aufgabe der vorliegenden Erfindung war es, Verbindungen zur Verfügung zu stellen, die als Substitutionstherapeutika bei Opiat-Abhängigkeit eingesetzt werden können und Vorteile gegenüber der Standardtherapie aufweisen.

Gegenstand der Erfindung sind daher ausgewählte Verbindungen aus der Gruppe der spirocyclischen Cyclohexan-Derivate der allgemeinen Formel I 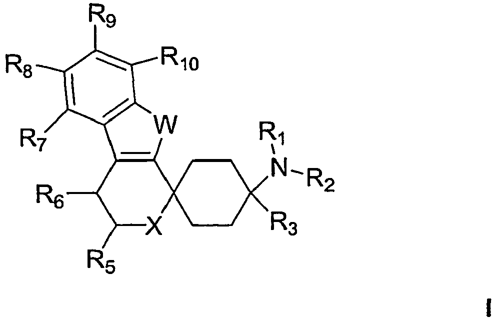 , worin
R¹ und R² unabhängig voneinander für H oder CH₃ stehen oder zusammen mit (CH₂)₄ oder (CH₂)₅ einen Ring bilden; wobei R¹ und R² nicht gleichzeitig H bedeuten;
R³ für Phenyl, Benzyl oder Heteroaryl jeweils unsubstituiert oder einfach oder mehrfach mit F, Cl, OH, CN oder OCH₃ substituiert, steht;
W für NR⁴, O oder S steht
   und
   R⁴ für H; C₁₋₅-Alkyl, Phenyl; über eine C₁₋₃-Alkyl-Gruppe gebundenes Phenyl, COR¹²; SO₂R¹² steht,
   wobei R¹² H; C₁₋₇-Alkyl, verzweigt oder unverzweigt, gesättigt oder ungesättigt, unsubstituiert oder einfach oder mehrfach mit OH, F oder COOC₁₋₄-Alkyl substituiert; C₄₋₆-Cycloalkyl; Aryl-, oder Heteroaryl, unsubstituiert oder einfach oder mehrfach mit F, Cl, Br, CF₃, OCH₃, C₁₋₄-Alkyl, verzweigt oder unverzweigt, substituiert; oder über gesättigtes oder ungesättigtes C₁₋₃-Alkyl gebundenes Phenyl oder Heteroaryl, unsubstituiert oder einfach oder mehrfach mit F, Cl, Br, CF₃, OCH₃, C₁₋₄-Alkyl, verzweigt oder unverzweigt, substituiert; oder über gesättigtes oder ungesättigtes C₁₋₃-Alkyl gebundenes C₅₋₆-Cycloalkyl; OR¹³; NR¹⁴R¹⁵ bedeutet;
R⁵ für H; COOR¹³, CONR¹³, OR¹³; C₁₋₅-Alkyl, gesättigt oder ungesättigt, verzweigt oder unverzweigt, unsubstituiert oder einfach oder mehrfach mit OH, F, CF₃ oder CN substituiert, steht;
R⁶ für H steht;
oder R⁵ und R⁶ gemeinsam (CH₂)ₙ mit n = 2, 3, 4, 5 oder 6 bedeuten, wobei einzelne Wasserstoffatome auch durch F, Cl, NO₂, CF₃, OR¹³, CN oder C₁₋₅-Alkyl ersetzt sein können;
R⁷, R⁸, R⁹ und R¹⁰ unabhängig voneinander für H, F, Cl, Br, NO₂, CF₃, OH, OCH3, CN, COOR¹³, NR¹⁴R¹⁵; C₁₋₅-Alkyl, Heteroaryl, unsubstituiert oder einfach oder mehrfach mit Benzyl, CH₃, Cl, F, OCH₃ oder OH substituiert, stehen;
   wobei R¹³ H oder C₁₋₅-Alkyl bedeutet;
   R¹⁴ und R¹⁵ unabhängig voneinander H oder C₁₋₅-Alkyl bedeuten;
X für O, S, SO, SO₂ oder NR¹⁷ steht;
   R¹⁷ für H; C₁₋₅-Alkyl, gesättigt oder ungesättigt, verzweigt oder unverzweigt; COR¹² oder SO₂R¹² steht,
in Form ihrer reinen Diastereomeren, ihrer Racemate, ihrer reinen Enantiomeren, oder in Form von Mischungen der Stereoisomeren in einem beliebigen Mischungsverhältnis;
   als Basen oder in Form ihrer Salze, insbesondere der physiologisch verträglichen Salze oder Salze physiologisch verträglicher Säuren oder Kationen; oder in Form ihrer Solvate,
wobei das Verhältnis der ORL1-Rezeptor-Affinität zur µ-Opioidrezeptor-Affnität 1,7 oder größer ist, zur Herstellung eines Arzneimittels zur Behandlung von Substanzabhängigkeit.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Hippursäure und/oder Asparaginsäure.

Die Ausdrücke "C₁₋₃-Alkyl" "C₁₋₅-Alkyl", "C₁₋₇-Alkyl" und "C₁₋₄-Alkyl" umfassen im Sinne dieser Erfindung acyclische gesättigte oder ungesättigte Kohlenwasserstoffreste, die verzweigt- oder geradkettig sein können, mit 1, 2 oder 3 C-Atomen bzw. 1, 2, 3, 4 oder 5 C-Atomen bzw 1, 2, 3, 4, 5, 6 oder 7 C-Atomen bzw. 1, 2, 3 oder 4 C-Atomen. Ungesättigte Verbindungen weisen mindestens eine C-C-Doppelbindung oder mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl; Ethylenyl (Vinyl), Ethinyl, Propenyl (-CH₂CH=CH₂, -CH=CH-CH₃, -C(=CH₂)-CH₃), Propinyl (-CH-C≡CH, -C≡C-CH₃), 1,1-Dimethylethyl, 1,1-Dimethylpropyl, Butenyl, Butinyl, Pentenyl und Pentinyl umfasst.

Der Ausdruck "C₅₋₆-Cycloalkyl" bedeutet für die Zwecke dieser Erfindung cyclische Kohlenwasserstoffe mit 5 oder 6 Kohlenstoffatomen, wobei die Kohlenwasserstoffe gesättigt oder ungesättigt (aber nicht aromatisch) sein können. Vorteilhaft ist C₅₋₆₋Cycloalkyl aus der Gruppe ausgewählt, die Cyclopentyl und Cyclohexyl umfasst.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Furyl (Furanyl), Benzofuranyl, Thienyl, Pyrazolyl, Imidazolyl, Thiazolyl, Oxazolyl, Isoxazolyl, Pyridinyl, oder Carbazolyl enthält, wobei die Bindung an die Verbindungen der allgemeinen Struktur I über jedes beliebige und mögliche Ringglied des Heteroaryl-Restes erfolgen kann. Besonders bevorzugt sind Pyridyl und Thienyl. Ebenfalls besonders bevorzugt sind Pyrazolyl und Furyl.

Überaschenderweise haben tierexperimentelle Untersuchungen mit Substanzen aus WO 0443967 und PCT/EP2004/014539 ergeben, dass die Substanzen mit einer besonders hohen ORL-1-Affinität, die nicht mehr als eine Größenordnung niedriger ist als die µ-Opioid-Rezeptor-Affinität, in besonderem Maße für eine Opiatsubstitution geeignet sind und damit das Potential für eine wesentliche Verbesserung der Substitutionstherapie besitzen.

Innerhalb der in WO 0443967 und PCT/EP2004/014539 beschriebenen Spiroverbindungen gibt es Verbindungen mit höherer und niedrigerer ORL-1-Komponente. Im Naloxon-induzierten Entzugsspringen in der Maus konnte gezeigt werden, dass spirocyclische Verbindungen mit einer ORL-1-Komponente, die nicht mehr als einen Faktor 10 schwächer ist als die µ-Komponente, Entzugsspringen unterdrücken. Verbindungen mit einer schwächeren ORL1-Komponente lösen dagegen Entzugsspringen aus. Im Test "Entzugsspringen" werden Mäuse über einen definierten Zeitraum hinweg mehrfach mit der Testsubstanz behandelt. Bei einem µ-Opioid wird innerhalb dieses Zeitraumes eine körperliche Abhängigkeit erreicht. Am Ende der Behandlung wird durch Gabe von Naloxon, einem µ-Antagonisten, die Wirkung des Opioids mit einem Mal aufgehoben. Bei Ausprägung einer körperlichen Abhängigkeit zeigen die Mäuse charakteristische Entzugssymptome, die sich in Form von Sprungbewegungen äußern (Saelens JK, Arch Int Pharmacodyn 190: 213-218,1971).

Die erfindungsgemäßen Verbindungen besitzen auf Grund der ORL1-Wirkkomponente zusätzliche Eigenschaften, die die Standardsubstanzen wie Methadon, LAAM oder Buprenorphin nicht besitzen und die zur Therapieverbesserung führen. Im Entzugsspringen an der Maus wurde gezeigt, dass bei Tieren, die mit kombinierten µ-/ORL1-Agonisten wie Beispiel 1 , Beispiel 3 oder Beispiel 4 behandelt wurden, durch Naloxon kein oder ein nur minimales Entzugsverhalten ausgelöst wird. Dies belegt, dass durch die ORL1-Komponente die Entzugssymptomatik unterdrückt wird. Daraus ergibt sich ein wesentlicher Vorteil, wenn diese Substanzen in der Entzugsbehandlung eingesetzt werden, da das Auftreten von Entzugsymptomen eine der wichtigsten Nebenwirkungen dieser Therapie darstellt und häufig zum Therapieabbruch führt. Das Verhältnis ORL1/µ-Affinität sollte 1,7 oder größer sein.

Das Ergebnis, dass die erfindungsgemäßen Verbindungen mit einer stärkeren ORL-1-Komponente das Entzugsspringen unterdrücken, das durch die µ-Komponente ausgelöst werden sollte, zeigt die Wirksamkeit dieser Verbindungen für die Verwendung in der Substitutionstherapie. Es ist daher bevorzugt, dass es sich bei der Substanzabhängigkeit um Opiatabhängigkeit handelt, beispielsweise von Alfentanil, Buprenorphin, Butorphanol, Codein, Dextromoramid, Dextropropoxyphen, Dezocin, Dihydrocodein, Diphenoxylat, Ethylmorphin, Hydrocodon, Hydromorphon, Ketobemidon, LAAM, Levorphanol, Meptazinol, Oxycodon, Oxymorphone, Fentanyl, Morphin, Heroin, Pethidin, Sufentanil oder Tilidin, vorzugsweise von Morphin, Methadon oder Heroin.

Die erfindungsgemäßen spirocyclischen Cyclohexanderivate können auch in Kombination mit einem Opioid-Rezeptor-Antagonisten eingesetzt werden, vorzugsweise mit Naloxon.

Bei der Synthese der erfindungsgemäßen Verbindungen, wie sie in WO 0443967 und PCT/EP2004/014539 beschrieben ist, können zwei oder mehr Diastereomere anfallen. In den meisten Fällen weisen die unpolareren Diastereomeren im Vergleich zu den polareren Diastereomeren eine höhere Affinität zu den beiden untersuchten Opiatrezeptoren auf. Daher ist es bevorzugt, dass die erfindungsgemäßen Substanzen in der Form des unpolareren Diastereomer vorliegen.

Ausgewählt im Sinne dieser Erfindung sind spirocyclische Cyclohexanderivate, worin R³ für Phenyl, unsubstituiert oder mit F, OH, Cl oder OCH₃ einfach oder mehrfach substituiert, oder Benzyl steht.

Weiterhin ausgewählt sind spirocyclische Cyclohexanderivate, worin R¹ und R² für CH₃ stehen

Die augemeine Formel I umfasst spirocyclische Cyclohexanderivaten aus der Gruppe

| | |
|---|---|
| 1 | 1,1-(3-Dimethylamino-3-phenylpentamethylen)-1,3,4,9-tetrahydropyrano[3,4-b]indol |
| 2 | 1,1-(3-Dimethylamino-3-benzylpentamethylen)-2-(2-nitrobenzoyl)-1,3,4,9-tetrahydro-2,9-diazafluoren |
| 3 | 1,1-(3-Dimethylamino-3-phenylpentamethylen)-6-fluoro-1,3,4,9-tetrahydropyrano[3,4-b]indol |
| 4 | 1, 1-(3-Dimethylamino-3-(2-thienyl)pentamethylen)-1,3,4,9-tetrahydropyrano[3,4-b]indol |
| 5 | 1,1-(3-Dimethylamino-3-phenylpentamethylen)-3-methyl-1, 3,4,9-tetrahydropyrano[3,4-b]indol |
| 6 | 1,1-(3-Dimethylamino-3-phenylpentamethylen)-3,6-dimethyl-1,3,4,9-tetrahydropyrano[3,4-b]indol |
| 7 | 1,1-(3-Dimethylamino-3-(4-fluorophenyl)pentamethylen)--1,3,4,9-tetrahydro-pyrano[3,4-b]indol |
| 8 | 1,1-(3-Dimethylamino-3-phenylpentamethylen)-3-hydroxymethyl-1,3,4,9-tetrahydro-2,9-diazafluoren |
| 9 | 1,1-(3-Dimethylamino-3-phenylpentamethylen)-2-(2,3,4,5,6-pentafluorobenzoyl)-1,3,4,9-tetrahydro-2,9-diazafluoren |
| 10 | 1,1-(3-Dimethylamino-3-phenylpentamethylen)-2-(methoxycarbonyl)-1,3,4,9-tetrahydro-2,9-diazafluoren |
| 11 | 1,1-(3-Dimethylamino-3-phenylpentamethylen)-7-fluoro-1,3,4,9-tetrahydro-2,9-diazafluoren |
| 12 | 1, 1-(3-Dimethylamino-3-phenylpentamethylen)-1,3,4,9-tetrahydro-2-aza-9-oxafluoren |
| 13 | 1,1-(3-Dimethylamino-3-(3-fluorophenyl)pentamethylen)-1,3,4,9-tetrahydro-2,9-diazafluoren |
| 14 | 1,1-(3-Dimethylamino-3-phenylpentamethylen)-2-acetyl-1,3,4,9-tetrahydro-2,9-diazafluoren |
| 15 | 1,1-(3-Dimethylamino-3-(3-fluorophenyl)pentamethylen)-1,3,4,9-tetrahydro-pyrano[3,4-b]indol |
| 16 | 1,1-(3-Dimethylamino-3-phenylpentamethylen)-6-chloro-3-methyl-1,3,4,9-tetrahydropyrano[3,4-b]indol |
| 17 | 1,1-(3-Dimethylamino-3-(2-pyridyl)pentamethylen)-1,3,4,9-tetrahydro-2,9-diazafluoren |
| 18 | 1,1-(3-Dimethylamino-3-(3-thienyl)pentamethylen)-6-fluoro-1,3,4,9-tetrahydropyrano[3,4-b]indol |
| 19 | 1,1-(3-Dimethylamino-3-phenylpentamethylen)-2-acetyl-7-fluoro-1,3,4,9-tetrahydro-2,9-diazafluoren |
| 20 | 1,1-(3-Dimethylamino-3-phenylpentamethylen)-1,3,4,9-tetrahydro-2,9-diazafluoren |
| 21 | 1,1-(3-Dimethylamino-3-phenylpentamethylen)-3-methyl-1,3,4,9-tetra-hydropyrano[3,4-b]indol |
| 22 | 1,1-(3-Dimethylamino-3-phenylpentamethylen)-1,3,4,9-tetrahydro-2-thia-9-azafluoren |
| 23 | 1,1-(3-Dimethylamino-3-phenylpentamethylen)-3-methyl-6-nitro-1,3,4,9-tetrahydropyrano[3,4-b]indol |
| 24 | 1,1-(3-Dimethylamino-3-(3-thienyl)pentamethylen)-1,3,4,9-tetrahydropyrano[3,4-b]indol |
| 25 | 1,1-(3-Dimethylamino-3-(4-fluorophenyl)pentamethylen)-1,3,4,9-tetrahydro-2,9-diazafluoren |
| 26 | 1,1-(3-Dimethylamino-3-phenylpentamethylen)-3-methyl-1,3,4,9-tetrahydro-2,9-diazafluoren |
| 27 | 1,1-(3-Dimethylamino-3-phenylpentamethylen)-2-oxo-1,3,4,9-tetrahydro-2-thia-9-azafluoren |
| 28 | 1, 1-(3-Dimethylamino-3-phenylpentamethylen)-1,3,4,9-tetrahydro-2-thia-9-azafluoren |
| 29 | 1,1-(3-Dimethylamino-3-(2-thienyl)pentamethylen)-6-fluoro-1,3,4,9-tetrahydro-pyrano[3,4-b]indol |
| 30 | 1,1-(3-Dimethylamino-3-phenylpentamethylen)-3-methyl-1,3,4,9-tetrahydro-2,9-diazafluoren |
| 31 | 1,1-(3-Dimethylamino-3-phenylpentamethylen)-6-fluoro-3-methyl-1,3,4,9-tetrahydropyrano[3,4-b]indol |
| 32 | 1,1-(3-Dimethylamino-3-phenylpentamethylen)-6-hydroxy-1,3,4,9-tetrahydropyrano[3,4-b]indol |
| 33 | 1,1-(3-Dimethylamino-3-(3-hydroxyphenyl)pentamethylen)-1,3,4,9-tetrahydro-pyrano[3,4-b]indol |
| 34 | 1,1-(3-Dimethylamino-3-phenylpentamethylen)-1,3,4,9-tetrahydro-2-thia-9-azafluoren |
| 35 | 6,6-(3-Dimethylamino-3-phenylpentamethylen)-1,2,3,4,4a,6,7,11c-octahydro-5-oxa-7-aza-benzo[c]fluoren |
| 36 | 1,1-(3-Dimethylamino-3-phenylpentamethylen)-6-methyl-1,3,4,9-tetrahydropyrano[3,4-b]indol |
| 37 | 1,1-(3-Dimethylamino-3-(4-fluorophenyl)pentamethylen)-2-acetyl-1,3,4,9-tetrahydro-2,9-diazafluoren |
| 38 | 1,1-(3-Dimethylamino-3-benzylpentamethylen)-1,3,4,9-tetrahydro-2,9-diazafluoren |
| 39 | 1,1-(3-Dimethylamino-3-phenylpentamethylen)-6-fluoro-1,3,4,9-tetrahydro-2,9-diazafluoren |
| 40 | 1,1-(3-Dimethylamino-3-phenylpentamethylen)-6-methyl-1,3,4,9-tetrahydro-2,9-diazafluoren |
| 41 | 1,1-(3-Dimethylamino-3-phenylpentamethylen)-6-bromo-3-methyl-1,3,4,9-tetrahydropyrano[3,4-b]indol |
| 42 | 1,1-(3-Dimethylamino-3-phenylpentamethylen)-6-fluoro-1,3,4,9-tetrahydropyrano[3,4-b]indol |
| 43 | 1,1-(3-Dimethylamino-3-phenylpentamethylen)-6-bromo-1,3,4,9-tetrahydropyrano[3,4-b]indol |
| 44 | 1,1-(3-Dimethylamino-3-phenylpentamethylen)-6-methoxy-1,3,4,9-tetra-hydropyrano[3,4-b]indol |
| 45 | 1,1-(3-(1-Pyrrolidyl)-3-phenylpentamethylen)-1,3,4,9-tetrahydropyrano[3,4-b]indol |
| 46 | 1,1-(3-Dimethylamino-3-phenylpentamethylen)-7,8-dichloro-1,3,4,9-tetra-hydropyrano[3,4-b]indol |
| 47 | 1, 1 -(3-Dimethylamino-3-phenylpentamethylen)-6-(1 -benzyl-pyrazol-4-yl)-1,3,4,9-tetrahydropyrano[3,4-b]indol |
| 48 | 1,1-(3-Methylamino-3-(2-thienyl)pentamethylen)-1,3,4,9-tetrahyd ropyrano[3,4-b]indol |
| 49 | 1,1-(3-Methylamino-3-(2-thienyl)pentamethylen)-6-fluoro-1,3,4,9-tetrahydro-pyrano[3,4-b]indol |
| 50 | 1,1-(3-Dimethylamino-3-(3-fluorophenyl)pentamethylen)-2-cinnamoyl-1,3,4,9-tetrahydro-2,9-diazafluoren |
| 51 | 1,1-(3-Dimethylamino-3-(3-fluorophenyl)pentamethylen)-2-(3,3-dimethylbutanoyl)-1,3,4,9-tetrahydro-2,9-diazafluoren |
| 52 | 1,1-(3-Dimethylamino-3-(3-fluorophenyl)pentamethylen)-2-(cyclopentylcarbonyl)-1,3,4,9-tetrahydro-2,9-diazafluoren |
| 53 | 1,1-(3-Dimethylamino-3-phenylpentamethylen)-2-(3,3-dimethylbutanoyl)-1,3,4,9-tetrahydro-2,9-diazafluoren |
| 54 | 1,1-(3-Dimethylamino-3-(3-fluorophenyl)pentamethylen)-2-(pentanoyl)-1,3,4,9-tetrahydro-2,9-diazafluoren |
| 55 | 1,1-(3-Dimethylamino-3-(3-fluorophenyl)pentamethylen)-2-(2-furylcarbonyl)-1,3,4,9-tetrahydro-2,9-diazafluoren |
| 56 | 1,1-(3-Dimethylamino-3-phenylpentamethylen)-2-(2-furylcarbonyl)-1,3,4,9-tetrahydro-2,9-diazafluoren |
| 57 | 1, 1 -(3-Dimethylamino-3-(3-fluorophenyl)pentamethylen)-2-(4-fluorobenzoyl)-1,3,4,9-tetrahydro-2,9-diazafluoren |
| 58 | 1, 1-(3-Dimethylamino-3-(3-fluorophenyl)pentamethylen)-2-(2-methoxybenzoyl)-1,3,4,9-tetrahydro-2,9-diazafluoren |
| 59 | 1,1-(3-Dimethylamino-3-phenylpentamethylen)-2-(3-methylbutanoyl)-1,3,4,9-tetrahydro-2,9-diazafluoren |
| 60 | 1,1-(3-Dimethylamino-3-(3-fluorophenyl)pentamethylen)-2-(cyclohexylcarbonyl)-1,3,4,9-tetrahydro-2,9-diazafluoren |
| 61 | 1,1-(3-Dimethylamino-3-(3-fluorophenyl)pentamethylen)-2-benzoyl-1,3,4,9-tetrahydro-2,9-diazafluoren |
| 62 | 1,1-(3-Dimethylamino-3-phenylpentamethylen)-2-(cyclohexylcarbonyl)-1,3,4,9-tetrahydro-2,9-diazafluoren |
| 63 | 1,1-(3-Dimethylamino-3-(3-fluorophenyl)pentamethylen)-2-(phenylacetyl)-1,3,4,9-tetrahydro-2,9-diazafluoren |
| 64 | 1,1-(3-Dimethylamino-3-(3-fluorophenyl)pentamethylen)-2-(4-methoxybenzoyl)-1,3,4,9-tetrahydro-2,9-diazafluoren |
| 65 | 1, 1-(3-Dimethylamino-3-phenylpentamethylen)-2-(4-trifluoromethylbenzoyl)-1,3,4,9-tetrahydro-2,9-diazafluoren |
| 66 | 1, 1 -(3-Dimethylamino-3-(3-fluorophenyl)pentamethylen)-2-(thien-2-ylacetyl)-1,3,4,9-tetrahydro-2,9-diazafluoren |
| 67 | 1,1-(3-Dimethylamino-3-(3-fluorophenyl)pentamethylen)-2-(phenylacetyl)-1,3,4,9-tetrahydro-2,9-diazafluoren |
| 68 | 1,1-(3-Dimethylamino-3-(3-fluorophenyl)pentamethylen)-2-butanoyl-1,3,4,9-tetrahydro-2,9-diazafluoren |
| 69 | 1,1-(3-Dimethylamino-3-benzylpentamethylen)-2-(4-trifluoromethylbenzoyl)-1,3,4,9-tetrahydro-2,9-diazafluoren |
| 70 | 1,1-(3-Dimethylamino-3-benzylpentamethylen)-2-(3-fluoro-5-trifluoromethylbenzoyl)-1,3,4,9-tetrahydro-2,9-diazafluoren |
| 71 | 1,1-(3-Dimethylamino-3-(3-fluorophenyl)pentamethylen)-2-(2,3-difluorobenzoyl)-1,3,4,9-tetrahydro-2,9-diazafluoren |
| 72 | 1,1-(3-Dimethylamino-3-(3-fluorophenyl)pentamethylen)-2-(4-methylbenzoyl)-1,3,4,9-tetrahydro-2,9-diazafluoren |
| 73 | 1,1-(3-Dimethylamino-3-phenylpentamethylen)-2-(3-phenylpropanoyl)-1,3,4,9-tetrahydro-2,9-diazafluoren |
| 74 | 1,1-(3-Dimethylamino-3-phenylpentamethylen)-2-(3,4-dichlorobenzoyl)-1,3,4,9-tetrahydro-2,9-diazafluoren |
| 75 | 1,1-(3-Dimethylamino-3-benzylpentamethylen)-2-cinnamoyl-1,3,4,9-tetrahydro-2,9-diazafluoren |
| 76 | 1,1-(3-Dimethylamino-3-benzylpentamethylen)-2-(2,6-difluorobenzoyl)-1,3,4,9-tetrahydro-2,9-diazafluoren |
| 77 | 1,1-(3-Dimethylamino-3-phenylpentamethylen)-2-(3-methoxycarbonylpropanoyl)-1,3,4,9-tetrahydro-2,9-diazafluoren |
| 78 | 1,1-(3-Dimethylamino-3-(3-fluorophenyl)pentamethylen)-2-(3-chlorobenzoyl)-1,3,4,9-tetrahydro-2,9-diazafluoren |
| 79 | 1,1-(3-Dimethylamino-3-phenylpentamethylen)-2-(3-fluorobenzoyl)-1,3,4,9-tetrahydro-2,9-diazafluoren |
| 80 | 1,1-(3-Dimethylamino-3-phenylpentamethylen)-2-(2,6-difluorobenzoyl)-1,3,4,9-tetrahydro-2,9-diazafluoren |
| 81 | 1,1-(3-Dimethylamino-3-(3-fluorophenyl)pentamethylen)-2-(3,4-dichlorobenzoyl)-1,3,4,9-tetrahydro-2,9-diazafluoren |
| 82 | 1,1-(3-Dimethylamino-3-benzylpentamethylen)-2-(2,3,4,5,6-pentafluorobenzoyl)-1,3,4,9-tetrahydro-2,9-diazafluoren |
| 83 | 1,1-(3-Dimethylamino-3-benzylpentamethylen)-2-cyclohexylcarbonyl-1,3,4,9-tetrahydro-2,9-diazafluoren |
| 84 | 1,1-(3-Dimethylamino-3-phenylpentamethylen)-2-(3-cyclopentylpropanoyl)-1,3,4,9-tetrahydro-2,9-diazafluoren |
| 85 | 1,1-(3-Dimethylamino-3-(3-fluorophenyl)pentamethylen)-2-(4-fluoro-3-trifluoromethyl-benzoyl)-1,3,4,9-tetrahydro-2,9-diazafluoren |
| 86 | 1,1-(3-Dimethylamino-3-(3-fluorophenyl)pentamethylen)-2-(4-methylbenzoyl)-1,3,4,9-tetrahydro-2,9-diazafluoren |
| 87 | 1,1-(3-Dimethylamino-3-(3-fluorophenyl)pentamethylen)-2-(3,4,5-trimethoxybenzoyl)-1,3,4,9-tetrahydro-2,9-diazafluoren |
| 88 | 1,1-(3-Dimethylamino-3-benzylpentamethylen)-2-cyclobutylcarbonyl-1,3,4,9-tetrahydro-2,9-diazafluoren |
| 89 | 1,1-(3-Dimethylamino-3-benzylpentamethylen)-2-(2,6-dichlorobenzoyl)-1,3,4,9-tetrahydro-2,9-diazafluoren |
| 90 | 1,1-(3-Dimethylamino-3-(3-fluorophenyl)pentamethylen)-2-(3-methoxybenzoyl)-1,3,4,9-tetrahydro-2,9-diazafluoren |
| 91 | 1,1-(3-Dimethylamino-3-phenylpentamethylen)-2-(2-thienylcarbonyl)-1,3,4,9-tetrahydro-2,9-diazafluoren |
| 92 | 1,1-(3-Dimethylamino-3-(3-fluorophenyl)pentamethylen)-2-propanoyl-1,3,4,9-tetrahydro-2,9-diazafluoren |
| 93 | 1,1-(3-Dimethylamino-3-(3-fluorophenyl)pentamethylen)-2-(2,4-difluorobenzoyl)-1,3,4,9-tetrahydro-2,9-diazafluoren |
| 94 | 1,1-(3-Dimethylamino-3-(3-fluorophenyl)pentamethylen)-2-(3,5-difluorobenzoyl)-1,3,4,9-tetrahydro-2,9-diazafluoren |
| 95 | 1,1-(3-Dimethylamino-3-(3-fluorophenyl)pentamethylen)-2-(4-tert.butylbenzoyl)-1,3,4,9-tetrahydro-2,9-diazafluoren |
| 96 | 1,1-(3-Dimethylamino-3-benzylpentamethylen)-2-(3-methoxycarbonylpropanoyl)-1,3,4,9-tetrahydro-2,9-diazafluoren |
| 97 | 1,1-(3-Dimethylamino-3-(3-fluorophenyl)pentamethylen)-2-(3,4-difluorobenzoyl)-1,3,4,9-tetrahydro-2,9-diazafluoren |
| 98 | 1,1-(3-Dimethylamino-3-phenylpentamethylen)-2-(2-ethylhexanoyl)-1,3,4,9-tetrahydro-2,9-diazafluoren |
| 99 | 1,1-(3-Dimethylamino-3-(3-fluorophenyl)pentamethylen)-2-(2-methylbenzoyl)-1,3,4,9-tetrahydro-2,9-diazafluoren |
| 100 | 1,1-(3-Dimethylamino-3-phenylpentamethylen)-2-(3-phenylpropanoyl)-1,3,4,9-tetrahydro-2,9-diazafluoren |
| 101 | 1,1-(3-Dimethylamino-3-(3-fluorophenyl)pentamethylen)-2-(3-fluoro-5-trifluoro-methylbenzoyl)-1,3,4,9-tetrahydro-2,9-diazafluoren |
| 102 | 1,1-(3-Dimethylamino-3-phenylpentamethylen)-2-(3-fluoro-5-trifluoro-methylbenzoyl)-1,3,4,9-tetrahydro-2,9-diazafluoren |
| 103 | 1,1-(3-Dimethylamino-3-(3-fluorophenyl)pentamethylen)-2-(4-nitrobenzoyl)-1,3,4,9-tetrahydro-2,9-diazafluoren |
| 104 | 1,1-(3-Dimethylamino-3-phenylpentamethylen)-2-(3,5-difluorobenzoyl)-1,3,4,9-tetrahydro-2,9-diazafluoren |
| 105 | 1,1-(3-Dimethylamino-3-benzylpentamethylen)-2-(2-trifluoromethylbenzoyl)-1,3,4,9-tetrahydro-2,9-diazafluoren |
| 106 | 1,1-(3-Dimethylamino-3-(3-fluorophenyl)pentamethylen)-2-(3,4-difluorobenzoyl)-1,3,4,9-tetrahydro-2,9-diazafluoren |
| 107 | 1,1-(3-Dimethylamino-3-phenylpentamethylen)-2-(4-chlorobenzoyl)-1,3,4,9-tetrahydro-2,9-diazafluoren |
| 108 | 1,1-(3-Dimethylamino-3-(3-fluorophenyl)pentamethylen)-2-(3-methylbenzoyl)-1,3,4,9-tetrahydro-2,9-diazafluoren |
| 109 | 1,1-(3-Dimethylamino-3-(3-fluorophenyl)pentamethylen)-2-(2,3,4,5-tetrafluorobenzoyl)-1,3,4,9-tetrahydro-2,9-diazafluoren |
| 110 | 1,1-(3-Dimethylamino-3-(3-fluorophenyl)pentamethylen)-2-(2,6-difluorobenzoyl)-1,3,4,9-tetrahydro-2,9-diazafluoren |
| 111 | 1,1-(3-Dimethylamino-3-phenylpentamethylen)-2-(2-furylcarbonyl)-1,3,4,9-tetrahydro-2,9-diazafluoren |
| 112 | 1,1-(3-Dimethylamino-3-(3-fluorophenyl)pentamethylen)-2-(2,4-dichlorobenzoyl)-1,3,4,9-tetrahydro-2,9-diazafluoren |
| 113 | 1,1-(3-Dimethylamino-3-(3-fluorophenyl)pentamethylen)-2-(3-methoxycarbonylpropanoyl)-1,3,4,9-tetrahydro-2,9-diazafluoren |
| 114 | 1,1-(3-Dimethylamino-3-(3-fluorophenyl)pentamethylen)-2-(3-chlorobenzoyl)-1,3,4,9-tetrahydro-2,9-diazafluoren |
| 115 | 1,1-(3-Dimethylamino-3-(3-fluorophenyl)pentamethylen)-2-(2-fluoro-5-trifluoromethylbenzoyl)-1,3,4,9-tetrahydro-2,9-diazafluoren |
| 116 | 1, 1 -(3-Dimethylamino-3-(3-fluorophenyl)pentamethylen)-2-(2-fluoro-4-trifluoro-methylbenzoyl)-1,3,4,9-tetrahydro-2,9-diazafluoren |
| 117 | 1,1-(3-Dimethylamino-3-benzylpentamethylen)-2-(2-thienylacetyl)-1,3,4,9-tetrahydro-2,9-diazafluoren |
| 118 | 1,1-(3-Dimethylamino-3-benzylpentamethylen)-2-(2,6-dichlorobenzoyl)-1,3,4,9-tetrahydro-2,9-diazafluoren |
| 119 | 1,1-(3-Dimethylamino-3-phenylpentamethylen)-2-(3,4-difluorobenzoyl)-1,3,4,9-tetrahydro-2,9-diazafluoren |
| 120 | 1,1-(3-Dimethylamino-3-benzylpentamethylen)-2-(3-methylbenzoyl)-1,3,4,9-tetrahydro-2,9-diazafluoren |
| 121 | 1,1-(3-Dimethylamino-3-phenylpentamethylen)-2-(3-methoxycarbonylpropanoyl)-1,3,4,9-tetrahydro-2,9-diazafluoren |
| 122 | 1,1-(3-Dimethylamino-3-(3-fluorophenyl)pentamethylen)-2-(2-fluoro-6-trifluoromethylbenzoyl)-1,3,4,9-tetrahydro-2,9-diazafluoren |
| 123 | 1,1-(3-Dimethylamino-3-phenylpentamethylen)-2-(2-methylbenzoyl)-1,3,4,9-tetrahydro-2,9-diazafluoren |
| 124 | 1,1-(3-Dimethylamino-3-phenylpentamethylen)-2-(3,5-dichlorobenzoyl)-1,3,4,9-tetrahydro-2,9-diazafluoren |
| 125 | 1,1-(3-Dimethylamino-3-benzylpentamethylen)-2-(2-furylcarbonyl)-1,3,4,9-tetrahydro-2,9-diazafluoren |
| 126 | 1,1-(3-Dimethylamino-3-benzylpentamethylen)-2-(pentanoyl)-1,3,4,9-tetrahydro-2,9-diazafluoren |
| 127 | 1,1-(3-Dimethylamino-3-benzylpentamethylen)-2-(6-fluoro-2-trifluoromethylbenzoyl)-1,3,4,9-tetrahydro-2,9-diazafluoren |
| 128 | 1,1-(3-Dimethylamino-3-(3-fluorophenyl)pentamethylen)-2-(3-methoxybenzoyl)-1,3,4,9-tetrahydro-2,9-diazafluoren |
| 129 | 1,1-(3-Dimethylamino-3-benzylpentamethylen)-2-(4-tert.butylbenzoyl)-1,3,4,9-tetrahydro-2,9-diazafluoren |
| 130 | 1,1-(3-Dimethylamino-3-benzylpentamethylen)-2-(phenylacetyl)-1,3,4,9-tetrahydro-2,9-diazafluoren |
| 131 | 1,1-(3-Dimethylamino-3-(3-fluorophenyl)pentamethylen)-2-(3,5-dichlorobenzoyl)-1,3,4,9-tetrahydro-2,9-diazafluoren |
| 132 | 1,1-(3-Dimethylamino-3-benzylpentamethylen)-2-(2,3,4,5,6-pentafluorobenzoyl)-1,3,4,9-tetrahydro-2,9-diazafluoren |
| 133 | 1,1-(3-Dimethylamino-3-benzylpentamethylen)-2-(3-nitrobenzoyl)-1,3,4,9-tetrahydro-2,9-diazafluoren |
| 134 | 1,1-(3-Dimethylamino-3-phenylpentamethylen)-2-(3-nitrobenzoyl)-1,3,4,9-tetrahydro-2,9-diazafluoren |
| 135 | 1,1-(3-Dimethylamino-3-benzylpentamethylen)-2-(2,3,4,5-tetrafluorobenzoyl)-1,3,4,9-tetrahydro-2,9-diazafluoren |
| 136 | 1,1-(3-Dimethylamino-3-phenylpentamethylen)-2-(2-thienylacetyl)-1,3,4,9-tetrahyd ro-2,9-diazafluoren |
| 137 | 1,1-(3-Dimethylamino-3-phenylpentamethylen)-2-(2-fluoro-4-trifluoromethylbenzoyl)-1,3,4,9-tetrahydro-2,9-diazafluoren |
| 138 | 1,1-(3-Dimethylamino-3-phenylpentamethylen)-2-(2-fluorobenzoyl)-1,3,4,9-tetrahydro-2,9-diazafluoren |
| 139 | 1,1-(3-Dimethylamino-3-phenylpentamethylen)-2-(cyclobutylcarbonyl)-1,3,4,9-tetrahydro-2,9-diazafluoren |
| 140 | 1,1-(3-Dimethylamino-3-phenylpentamethylen)-2-(2-methoxybenzoyl)-1,3,4,9-tetrahydro-2,9-diazafluoren |
| 141 | 1,1-(3-Dimethylamino-3-phenylpentamethylen)-2-(2,5-difluorobenzoyl)-1,3,4,9-tetrahydro-2,9-diazafluoren |
| 142 | 1,1-(3-Dimethylamino-3-(3-fluorophenyl)pentamethylen)-2-(2-bromobenzoyl)-1,3,4,9-tetrahydro-2,9-diazafluoren |
| 143 | 1,1-(3-Dimethylamino-3-phenylpentamethylen)-2-(3-fluoro-5-trifluoromethylbenzoyl)-1,3,4,9-tetrahydro-2,9-diazafluoren |
| 144 | 1,1-(3-Dimethylamino-3-phenylpentamethylen)-2-(2-fluoro-5-trifluoromethylbenzoyl)-1,3,4,9-tetrahydro-2,9-diazafluoren |
| 145 | 1,1-(3-Dimethylamino-3-phenylpentamethylen)-2-(2,3-difluorobenzoyl)-1,3,4,9-tetrahydro-2,9-diazafluoren |

Ausgewählt im Rahme der vorliegenden Erfindung sind Verbindungen aus der Gruppe

| | |
|---|---|
| 50 | 1,1-(3-Dimethylamino-3-(3-fluorophenyl)pentamethylen)-2-cinnamoyl-1,3,4,9-tetrahydro-2,9-diazafluoren |
| 51 | 1,1-(3-Dimethylamino-3-(3-fluorophenyl)pentamethylen)-2-(3,3-dimethylbutanoyl)-1,3,4,9-tetrahydro-2,9-diazafluoren |
| 52 | 1,1-(3-Dimethylamino-3-(3-fluorophenyl)pentamethylen)-2-(cyclopentylcarbonyl)-1,3,4,9-tetrahydro-2,9-diazafluoren |
| 53 | 1,1-(3-Dimethylamino-3-phenylpentamethylen)-2-(3,3-dimethylbutanoyl)-1,3,4,9-tetrahydro-2,9-diazafluoren |
| 54 | 1,1-(3-Dimethylamino-3-(3-fluorophenyl)pentamethylen)-2-(pentanoyl)-1,3,4,9-tetrahydro-2,9-diazafluoren |
| 55 | 1,1-(3-Dimethylamino-3-(3-fluorophenyl)pentamethylen)-2-(2-furylcarbonyl)-1,3,4,9-tetrahydro-2,9-diazafluoren |
| 56 | 1,1-(3-Dimethylamino-3-phenylpentamethylen)-2-(2-furylcarbonyl)-1,3,4,9-tetrahydro-2,9-diazafluoren |
| 57 | 1,1-(3-Dimethylamino-3-(3-fluorophenyl)pentamethylen)-2-(4-fluorobenzoyl)-1,3,4,9-tetrahydro-2,9-diazafluoren |
| 58 | 1,1-(3-Dimethylamino-3-(3-fluorophenyl)pentamethylen)-2-(2-methoxybenzoyl)-1,3,4,9-tetrahydro-2,9-diazafluoren |
| 59 | 1,1-(3-Dimethylamino-3-phenylpentamethylen)-2-(3-methylbutanoyl)-1,3,4,9-tetrahydro-2,9-diazafluoren |
| 60 | 1,1-(3-Dimethylamino-3-(3-fluorophenyl)pentamethylen)-2-(cyclohexylcarbonyl)-1,3,4,9-tetrahydro-2,9-diazafluoren |
| 61 | 1,1-(3-Dimethylamino-3-(3-fluorophenyl)pentamethylen)-2-benzoyl-1,3,4,9-tetrahydro-2,9-diazafluoren |
| 62 | 1,1-(3-Dimethylamino-3-phenylpentamethylen)-2-(cyclohexylcarbonyl)-1,3,4,9-tetrahydro-2,9-diazafluoren |
| 63 | 1,1-(3-Dimethylamino-3-(3-fluorophenyl)pentamethylen)-2-(phenylacetyl)-1,3,4,9-tetrahydro-2,9-diazafluoren |
| 64 | 1,1-(3-Dimethylamino-3-(3-fluorophenyl)pentamethylen)-2-(4-methoxybenzoyl)-1,3,4,9-tetrahydro-2,9-diazafluoren |
| 65 | 1,1-(3-Dimethylamino-3-phenylpentamethylen)-2-(4-trifluoromethylbenzoyl)-1,3,4,9-tetrahydro-2,9-diazafluoren |
| 66 | 1,1-(3-Dimethylamino-3-(3-fluorophenyl)pentamethylen)-2-(thien-2-ylacetyl)-1,3,4,9-tetrahydro-2,9-diazafluoren |
| 67 | 1,1-(3-Dimethylamino-3-(3-fluorophenyl)pentamethylen)-2-(phenylacetyl)-1,3,4,9-tetrahydro-2,9-diazafluoren |
| 68 | 1,1-(3-Dimethylamino-3-(3-fluorophenyl)pentamethylen)-2-butanoyl-1,3,4,9-tetrahydro-2,9-diazafluoren |
| 69 | 1,1-(3-Dimethylamino-3-benzylpentamethylen)-2-(4-trifluoromethylbenzoyl)-1,3,4,9-tetra hydro-2,9-diazafluoren |
| 70 | 1,1-(3-Dimethylamino-3-benzylpentamethylen)-2-(3-fluoro-5-trifluoromethylbenzoyl)-1,3,4,9-tetrahydro-2,9-diazafluoren |
| 71 | 1,1-(3-Dimethylamino-3-(3-fluorophenyl)pentamethylen)-2-(2,3-difluorobenzoyl)-1,3,4,9-tetrahydro-2,9-diazafluoren |
| 72 | 1,1-(3-Dimethylamino-3-(3-fluorophenyl)pentamethylen)-2-(4-methylbenzoyl)-1,3,4,9-tetrahydro-2,9-diazafluoren |

Die Synthese der Verbindungen ist in WO 0443967 und PCT/EP2004/014539 beschrieben. Die Isolierung der erfindungsgemäßen Verbindungen durch Säulenchromatographie mit Kieselgel als stationärer Phase führt zu einer Auftrennung der unterschiedlich polaren Diastereomeren. Diese wurden aufgrund ihrer Laufzeit als "unpolareres" Diastereomer (kürzere Laufzeit) und "polareres" Diastereomer (längere Laufzeit) charakterisiert. Es wurde Kieselgel 60 (0,040-0,063 mm) der Firma E. Merck, Darmstadt, eingesetzt. Die dünnschichtchromatographischen Untersuchungen wurden mit HPTLC-Fertigplatten der Firma E. Merck, Darmstadt, durchgeführt. Als Laufmittel wurde beispielsweise Methanol/Ethylacetat in einem Verhältnis von 1:1, 2:1, 3:1 oder 4:1, vorzugsweise 3:1, Methanol/konz. Ammoniaklösung 99,5:0,5 oder Methanol/Ethylacetat/konz. Ammoniaklösung in einem Verhältnis von 66:33:0,5 eingesetzt.

Die erfindungsgemäßen Arzneimittel enthalten neben mindestens einer erfindungsgemäßen Verbindung gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße spirocyclische Cyclohexan-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen verzögert freisetzen. Die erfindungsgemäßen Verbindungen können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 1 mg/kg, bevorzugt 0,0001 bis 0,05 mg/kg wenigstens eines erfindungsgemäßen spirocyclischen Cyclohexan-Derivats appliziert.

Alle vorstehenden Formen der erfindungsgemäßen Arzneimittel können neben wenigstens einer erfindungsgemäßen Verbindung noch einen weiteren Wirkstoff, insbesondere einen Opioid-Antagonisten, vorzugsweise Naloxon, enthalten.

### Vergleichsuntersuchungen

Zum Vergleich wurde die Standardmedikation Levomethadon, die keine nennenswerte ORL-1-Komponente besitzt, im Entzugsspringen getestet. Levomethadon, das zu den Standardtherapeutika in der Substitutionstherapie gehört, induzierte ebenfalls Entzugsspringen.

Unter den in WO 0443967 und PCT/EP2004/014539 beschriebenen Verbindungen findet sich eine ganze Reihe von Substanzen mit einer geringen ORL-1-Komponente. Zwei typische Vertreter mit einer geringen ORL-1-Komponente (Verbindungen m und n) wurden ebenfalls im Entzugsspringen getestet und induzierten Entzugsspringen. Dies zeigt die Wichtigkeit der ORL-1-Komponente für die Eignung einer Substanz für die Substitutionstherapie innerhalb der Gruppe der in WO 0443967 und PCT/EP2004/014539 beschriebenen Verbindungen.

Zu den bei den Standardtherapeutika angestrebten und teilweise verwirklichten Eigenschaften gehören:
1. Hohe opioide Potenz
2. Volle intrinsische Aktivität
3. Lange Wirkdauer (aktive Metabolite wie beispielsweise bei LAAM sind in dieser Indikation sogar erwünscht!)
4. Langsamer Wirkeintritt bes. bei oraler Gabe (geringer Kick)
5. Geringe Kardiotoxizität

Während bei den bekanten Standardsubstanzen einige dieser Eigenschaften fehlen oder nur unzureichend ausgebildet sind, konnten für die erfindungsgemäßen Verbindungen alle geforderten Eigenschaften nachgewiesen werden.

Die erfindungsgemäßen Verbindungen zeigen deutlich verminderte cadiovaskuläre Nebenwirkungen im Vergleich zu Methadon und LAAM. Die cardiovaskulären Nebenwirkungen werden zurückgeführt auf eine Verzögerung der kardialen Repolarisation (manifestiert sich als QTc-Verlängerung im Oberflächen-EKG), die durch die Blockade eines speziellen Kaliumkanals (HERG) hervorgerufen wird (Kornick et al. Pain 2003, 105, 499-506). Sowohl Methadon als auch LAAM zeigen Wechselwirkungen mit dem HERG-Kanal (Jiesheng et al., Eur. J. Pharmacol. 2003, 458, 25-29; Katchman et al., J. Pharmacol. Exp. Ther. 2002, 303, 688-694). Im Zusammenhang mit der Verordnung von Orlaam (LAAM) wurde 2001 von der FDA vor den cardiovaskulären Effekte gewarnt (JAMA 2001, 285, 2705). Für die erfindungsgemäßen Verbindungen wurde beispielhaft gezeigt, dass die cardiovaskulären Nebenwirkungen im Vergleich zu Methadon und LAAM deutlich verbessert sind.

### Beispiele:

Innerhalb der Gruppe der in WO 0443967 und PCT/EP2004/014539 beschriebenen Spiroverbindungen gibt es Verbindungen mit höherer und niedrigerer ORL-1-Komponente.

### Messung der ORL1-Bindung

Die Cyclohexan-Derivate der allgemeinen Formel I wurden in einem Rezeptorbindungsassay mit ³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer Kᵢ-Wert in oder % Inhibition bei c=1 µM angegeben.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden substituierten substituierten Cyclohexyl-1,4-diamin -Derivates mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I IC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

In der folgenden Tabelle sind beispielhaft einige Verbindungen aufgeführt, die zwar sehr gut an den µ-Opiat-Rezeptor binden, jedoch eine niedrigere ORL1-Komponente besitzen:

| **Verbindung** | **Struktur** | **Verhältnis ORL1/µ berechnet als Ki(µ) / Ki(ORL1) (nur bei gemessenen Ki-Werten)** | **ORL-1 [1 µM], % Hemmung** | **µ-Opiat-Rezeptor [1 µM], % Hemmung** |
|---|---|---|---|---|
| a | 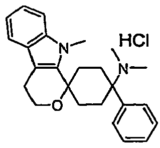 | | 1 | 83 |
| b | 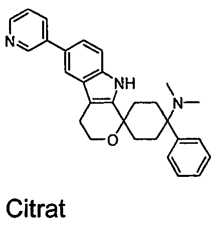 | | 5 | 89 |
| c | 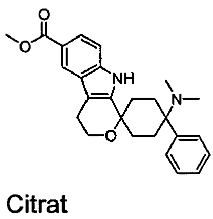 | | 11 | 74 |
| d | 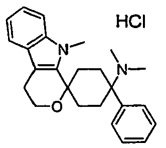 | | 13 | 83 |
| e | 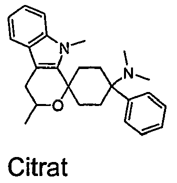 | | 15 | 75 |
| f | 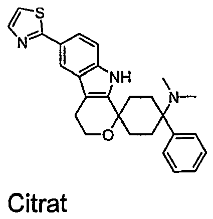 | | 27 | 92 |
| g | 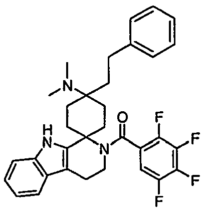 | | 31 | 91 |
| h | 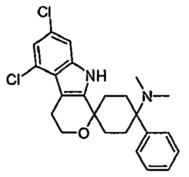 | | 32 | 97 |
| i | 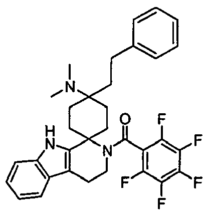 | | 32 | 99 |
| j | 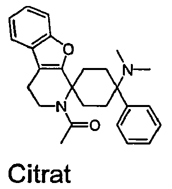 | | 33 | 78 |
| k | 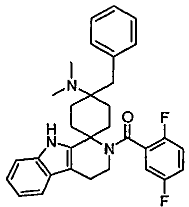 | | 41 | 82 |
| l | 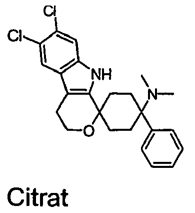 | | 43 | 100 |
| m | 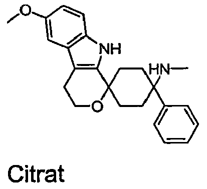 | 0,03 | 69 nM (Kₗ) | 2,4 nM (Kᵢ) |
| n | 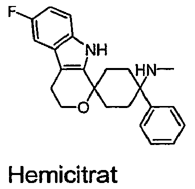 | 0,1 | 14,3 nM (Kᵢ) | 1,8 nM (Kᵢ) |

Wie beispielhaft an den Verbindungen m und n gezeigt, unterdrücken diese Verbindungen das Entzugsspringen nicht.

Im Vergleich dazu weisen die folgenden Verbindungen ein nahezu ausgeglichenes ORL1/µ-Verhältnis auf. Die erfindungsgemäßen Verbindungen besitzen eine stärker ausgeprägte ORL1-Komponente. Das Verhältnis ORL1:µ beträgt mindestens 1.7:

| Nr. | Verbindung * Vergleichsbeispiel | Name | Bemerkungen | Verhältnis ORL1/µ berechnet als Ki(µ) / Ki(ORL1) | ORL1 human Ki [µM] | µ Ki [µM] |
|---|---|---|---|---|---|---|
| 1* | 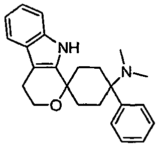 | 1,1-(3-Dimethylamino-3-phenylpentamethylen)-1,3,4,9-tetrahydro-pyrano[3,4-b]indol | Hemicitrat | 2 | 0,0034 | 0,0058 |
| | | | Unpolareres Diastereomer | | | |
| 2* | 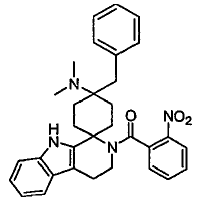 | 1,1-(3-Dimethylamino-3-benzylpentamethylen)-2-(2-nitrobenzoyl)-1,3,4,9-tetrahydro-2,9-diazafluoren | | 0,32 | 0,0038 | 0,0012 |
| 3* | 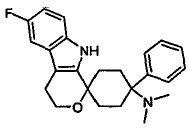 | 1,1-(3-Dimethylamino-3-phenylpentamethylen)-6-fluoro-1,3,4,9-tetrahydro-pyrano[3,4-b]indol | Hemicitrat | 2,45 | 0,0026 | 0,006 |
| | | | Unpolareres Diastereomer | | | |
| 4* | 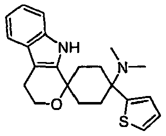 | 1,1-(3-Dimethylamino-3-(2-thienyl)pentamethylen)-1,3,4,9-tetrahydro-pyrano[3,4-b]indol | Hemicitrat | 2,07 | 0,0003 | 0,0006 |
| | | | Unpolareres Diastereomer | | | |
| 5* | 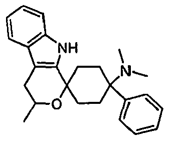 | 1,1-(3-Dimethylamino-3-phenylpentamethylen)-3-methyl-1,3,4,9-tetrahydropyrano[3,4-b]indol | Hemicitrat | 3,82 | 0,0012 | 0,0047 |
| | | | Unpolareres Diastereomer | | | |
| 6* | 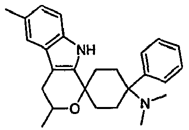 | 1,1-(3-Dimethylamino-3-phenylpentamethylen)-3,6-dimethyl-1,3,4,9-tetrahydropyrano[3,4-b]indol | Hemicitrat | 3,18 | 0,0044 | 0,014 |
| | | | Eins von zwei Diastereomeren | | | |
| 7* | 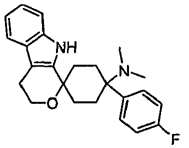 | 1,1-(3-Dimethylamino-3-(4-fluorophenyl)pentamethyl en)--1,3,4,9-tetrahydro-pyrano[3,4-b]indol | Hemicitrat | 3,00 | 0,0032 | 0,010 |
| | | | Eins von zwei Diastereomeren | | | |
| 8* | 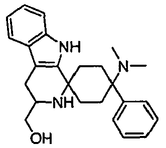 | 1,1-(3-Dimethylamino-3-phenylpentamethylen)-3-hydroxymethyl-1,3,4,9-tetrahydro-2,9-diazafluoren | Dicitrat | 2,70 | 0,0010 | 0,0027 |
| | | | Diastereomeren-gemisch | | | |
| 9* | 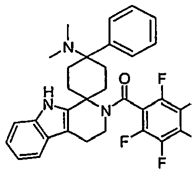 | 1,1-(3-Dimethylamino-3-phenylpentamethylen)-2-(2,3,4,5,6-pentafluoro-benzoyl)-1,3,4,9-tetrahydro-2,9-diazafluoren | | 0,32 | 0,13 | 0,041 |
| 10* | 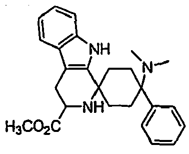 | 1,1-(3-Dimethylamino-3-phenylpentamethylen)-2-(methoxycarbonyl)-1,3,4,9-tetrahydro-2,9-diazafluoren | Citrat | 2,67 | 0,0009 | 0,0023 |
| | | | Diastereomeren-gemisch | | | |
| 11* | 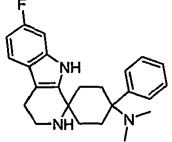 | 1,1-(3-Dimethylamino-3-phenylpentamethylen)-7-fluoro-1,3,4,9-tetrahydro-2,9-diazafluoren | Citrat | 2,46 | 0,007 | 0,02 |
| | | | Polareres Diastereomer | | | |
| 12* | 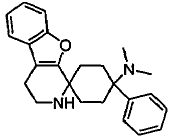 | 1,1-(3-Dimethylamino-3-phenylpentamethylen)-1,3,4,9-tetrahydro-2-aza-9-oxafluoren | Citrat | 2,39 | 0,07 | 0,17 |
| | | | Diastereomeren-gemisch | | | |
| 13* | 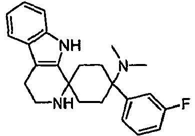 | 1,1-(3-Dimethylamino-3-(3-fluorophenyl)pentamethyl en)-1,3,4,9-tetrahydro-2,9-diazafluoren | Diastereomeren-gemisch | 2,22 | 0,0036 | 0,008 |
| 14* | 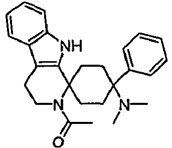 | 1,1-(3-Dimethylamino-3-phenylpentamethylen)-2-acetyl-1,3,4,9-tetrahydro-2,9-diazafluoren | Hydrochlorid | 1,98 | 0,0029 | 0,006 |
| | | | Unpolareres Diastereomer | | | |
| 15* | 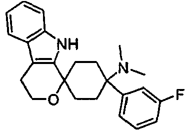 | 1,1-(3-Dimethylamino-3-(3-fluorophenyl)pentamethyl en)-1,3,4,9-tetrahydro-pyrano[3,4-b]indol | Hemicitrat | 1,79 | 0,0012 | 0,0022 |
| | | | Eins von zwei Diastereomeren | | | |
| 16* | 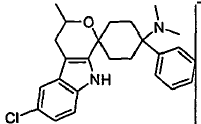 | 1,1-(3-Dimethylamino-3-phenylpentamethylen)-6-chloro-3-methyl-1,3,4,9-tetrahydropyrano[3,4-b]indol | Citrat | 1,77 | 0,06 | 0,11 |
| | | | Unpolareres Diastereomer | | | |
| 17* | 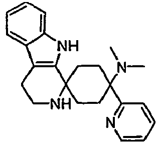 | 1,1-(3-Dimethylamino-3-(2-pyridyl)pentamethylen)-1,3,4,9-tetrahydro-2,9-diazafluoren | Diastereomenren -gemisch | 1,75 | 0,0016 | 0,0028 |
| 18* | 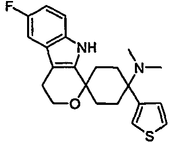 | 1,1-(3-Dimethylamino-3-(3-thienyl)pentamethylen)-6-fluoro-1,3,4,9-tetrahydropyrano[3,4-b]indol | Hemicitrat | 1,62 | 0,0003 | 0,0006 |
| | | | Unpolareres Diastereomer | | | |
| 19* | 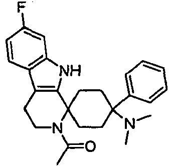 | 1,1-(3-Dimethylamino-3-phenylpentamethylen)-2-acetyl-7-fluoro-1,3,4,9-tetrahydro-2,9-diazafluoren | Citrat | 1,41 | 0,0011 | 0,0016 |
| | | | Diastereomenren -gemisch | | | |
| 20* | 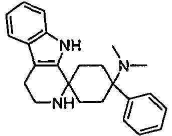 | 1,1-(3-Dimethylamino-3-phenylpentamethylen)-1,3,4,9-tetrahydro-2,9-diazafluoren | Dihydrochlorid | 1,38 | 0,0003 | 0,0004 |
| | | | Diastereomenren -gemisch | | | |
| 21* | 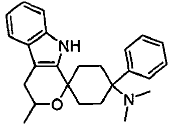 | 1,1-(3-Dimethylamino-3-phenylpentamethylen)-3-methyl-1,3,4,9-tetra-hydropyrano[3,4-b]indol | Citrat | 1,38 | 0,04 | 0,06 |
| | | | Diastereomenren -gemisch | | | |
| 22* | 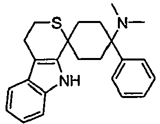 | 1,1-(3-Dimethylamino-3-phenylpentamethylen)-1,3,4,9-tetrahydro-2-thia-9-azafluoren | Citrat | 1,33 | 0,02 | 0,02 |
| | | | Diastereomenren -gemisch | | | |
| 23* | 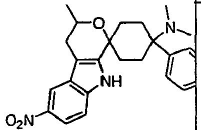 | 1,1-(3-Dimethylamino-3-phenylpentamethylen)-3-methyl-6-nitro-1,3,4,9-tetrahydropyrano[3,4-b]indol | Citrat | 1,29 | 0,03 | 0,04 |
| | | | Polareres Diastereomer | | | |
| 24* | 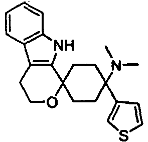 | 1,1-(3-Dimethylamino-3-(3-thienyl)pentamethylen)-1,3,4,9-tetrahydro-pyrano[3,4-b]indol | Hemicitrat | 1,19 | 0,0006 | 0,0007 |
| | | | Unpolareres Diastereomer | | | |
| 25* | 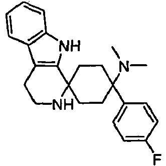 | 1,1-(3-Dimethylamino-3-(4-fluorophenyl)pentamethyl en)-1,3,4,9-tetrahydro-2,9-diazafluoren | Diastereomenren -gemisch | 1,12 | 0,0026 | 0,0029 |
| 26* | 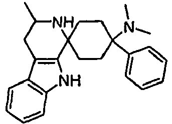 | 1,1-(3-Dimethylamino-3-phenylpentamethylen)-3-methyl-1,3,4,9-tetrahydro-2,9-diazafluoren | Citrat | 0,94 | 0,0005 | 0,0005 |
| | | | Eins von zwei Diastereomeren | | | |
| 27* | 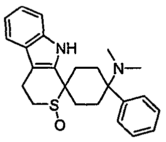 | 1,1-(3-Dimethylamino-3-phenylpentamethylen)-2-oxo-1,3,4,9-tetrahydro-2-thia-9-azafluoren | Citrat | 0,94 | 0,06 | 0,06 |
| 28* | 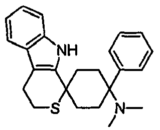 | 1,1-(3-Dimethylamino-3-phenylpentamethylen)-1,3,4,9-tetrahydro-2-thia-9-azafluoren | Hemicitrat | 0,84 | 0,0037 | 0,0031 |
| | | | Unpolareres Diastereomer | | | |
| 29* | 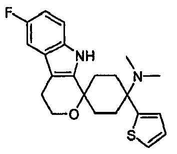 | 1,1-(3-Dimethylamino-3-(2-thienyl)pentamethylen)-6-fluoro-1,3,4,9-tetrahydro-pyrano[3,4-b]indol | Hemicitrat | 0,82 | 0,0007 | 0,0006 |
| | | | Unpolareres Diastereomer | | | |
| 30* | 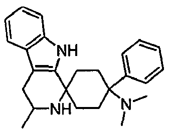 | 1,1-(3-Dimethylamino-3-phenylpentamethylen)-3-methyl-1,3,4,9-tetrahydro-2,9-diazafluoren | Dihydrochlorid | 0,78 | 0,0006 | 0,0005 |
| | | | Diastereomenren -gemisch | | | |
| 31* | 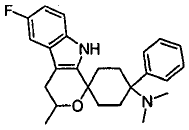 | 1,1-(3-Dimethylamino-3-phenylpentamethylen)-6-fluoro-3-methyl-1,3,4,9-tetrahydropyrano[3,4-b]indol | Hemicitrat | 0,73 | 0,08 | 0,06 |
| | | | Eins von zwei Diastereomeren | | | |
| 32* | 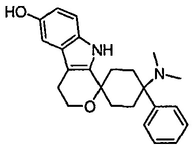 | 1,1-(3-Dimethylamino-3-phenylpentamethylen)-6-hydroxy-1,3,4,9-tetrahydropyrano[3,4-b]indol | Citrat | 0,7 | 0,0016 | 0,0011 |
| | | | Eins von zwei Diastereomeren | | | |
| 33* | 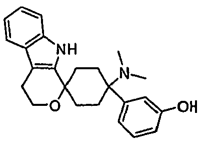 | 1,1-(3-Dimethylamino-3-(3-hydroxyphenyl)pentamet hylen)-1,3,4,9-tetrahydro-pyrano[3,4-b]indol | Methylsulfonat | 0,66 | 0,014 | 0,009 |
| | | | Eins von zwei Diastereomeren | | | |
| 34* | 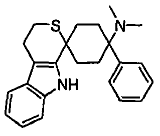 | 1,1-(3-Dimethylamino-3-phenylpentamethylen)-1,3,4,9-tetrahydro-2-thia-9-azafluoren | Citrat | 0,62 | 0,13 | 0,08 |
| | | | Polareres Diastereomer | | | |
| 35* | 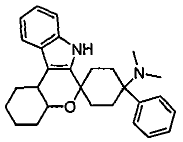 | 6,6-(3-Dimethylamino-3-phenylpentamethylen)-1,2,3,4,4a,6,7,11c-octahydro-5-oxa-7-aza-benzo[c]fluoren | Citrat | 0,62 | 0,11 | 0,07 |
| | | | Diastereomeren-gemisch | | | |
| 36* | 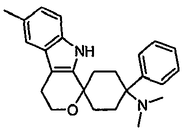 | 1,1-(3-Dimethylamino-3-phenylpentamethylen)-6- | Hemicitrat | 0,60 | 0,0045 | 0,0027 |
| | | methyl-1,3,4,9-tetrahydropyrano[3,4-b]indol | Eins von zwei Diastereomeren | | | |
| 37* | 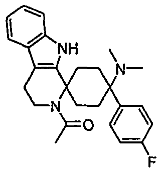 | 1,1-(3-Dimethylamino-3-(4- | Citrat | 0,55 | 0,006 | 0,0033 |
| | | fluorophenyl)pentamethyl en)-2-acetyl-1,3,4,9-tetrahydro-2,9-diazafluoren | Diastereomerengemisch | | | |
| 38* | 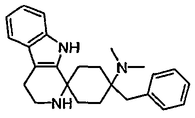 | 1,1-(3-Dimethylamino-3-benzylpentamethylen)-1,3,4,9-tetrahydro-2,9-diazafluoren | Eins von zwei Diastereomeren | 0,54 | 0,0011 | 0,0006 |
| 39* | 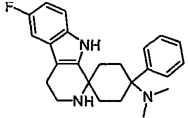 | 1,1-(3-D imethylamino-3-phenylpentamethylen)-6- | Dihydrochlorid | 0,51 | 0,0014 | 0,0007 |
| | | fluoro-1,3,4,9-tetrahydro-2,9-diazafluoren | Eins von zwei Diastereomeren | | | |
| 40* | 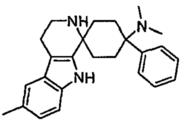 | 1,1-(3-Dimethylamino-3-phenylpentamethylen)-6-methyl-1,3,4,9-tetrahydro-2,9-diazafluoren | Citrat | 0,47 | 0,03 | 0,02 |
| | | | Polareres Diastereomer | | | |
| 41* | 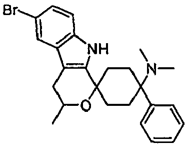 | 1,1-(3-Dimethylamino-3-phenylpentamethylen)-6-bromo-3-methyl-1,3,4,9-tetrahydropyrano[3,4-b]indol | Hemicitrat | 0,45 | 0,02 | 0,010 |
| | | | Eins von zwei Diastereomeren | | | |
| 42* | 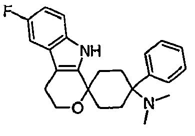 | 1,1-(3-Dimethylamino-3- | Citrat | 0,42 | 0,02 | 0,009 |
| | | phenylpentamethylen)-6-fluoro-1,3,4,9-tetrahydropyrano[3,4-b]indol | Polareres Diastereomer | | | |
| 43* | 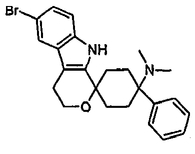 | 1,1-(3-Dimethylamino-3-phenylpentamethylen)-6-bromo-1,3,4,9-tetrahydropyrano[3,4-b]indol | Hemicitrat | 0,37 | 0,05 | 0,02 |
| | | | Eins von zwei Diastereomeren | | | |
| 44* | 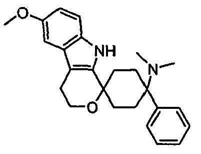 | 1,1-(3-Dimethylamino-3-phenylpentamethylen)-6-methoxy-1,3,4,9-tetra-hydropyrano[3,4-b]indol | Hydrochlorid | 0,34 | 0,006 | 0,0020 |
| | | | Unpolareres Diastereomer | | | |
| 45* | 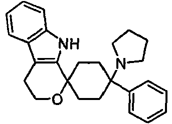 | 1,1-(3-(1-Pyrrolidyl)-3-phenylpentamethylen)-1,3,4,9-tetrahydro-pyrano[3,4-b]indol | Hemicitrat | 0,31 | 0,0035 | 0,0011 |
| | | | Unpolareres Diastereomer | | | |
| 46* | 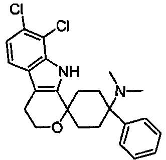 | 1,1-(3-Dimethylamino-3-phenylpentamethylen)-7,8-dichloro-1,3,4,9-tetra-hydropyrano[3,4-b]indol | Methansulfonat | 0,31 | 0,0045 | 0,0014 |
| | | | Eins von zwei Diastereomeren | | | |
| 47* | 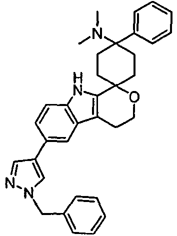 | 1,1-(3-Dimethylamino-3-phenylpentamethylen)-6-(1-benzyl-pyrazol-4-yl)-1,3,4,9-tetrahydro-pyrano[3,4-b]indol | Hydrochlorid | 2,73 | 0,0044 | 0,012 |
| | | | Unpolareres Diastereomer | | | |
| 48* | 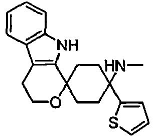 | 1,1-(3-Methylamino-3-(2-thienyl)pentamethylen)-1,3,4,9-tetrahydro-pyrano[3,4-b]indol | Eins von zwei Diastereomeren | 6,56 | 0,00032 | 0,0021 |
| 49* | 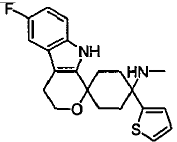 | 1,1-(3-Methylamino-3-(2-thienyl)pentamethylen)-6-fluoro-1,3,4,9-tetrahydro-pyrano[3,4-b]indol | Citrat | 0,7 | 0,0042 | 0,0028 |
| | | | Eins von zwei Diastereomeren | | | |

| Beispiel | Verbindung | Name | Verhältnis ORL1/µ | ORL1, Ki [µM] | N, .Ki [µM] |
|---|---|---|---|---|---|
| 50 | 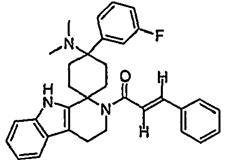 | 1,1-(3-Dimethylamino-3-(3-fluorophenyl)pentamethylen)-2-cinnamoyl-1,3,4,9-tetrahydro-2,9-diazafluoren | 21,43 | 0,0014 | 0,030000 |
| 51 | 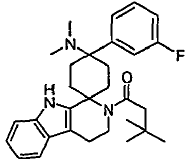 | 1,1-(3-Dimethylamino-3-(3-fluorophenyl)pentamethylen)-2-(3,3-dimethylbutanoyl)-1,3,4,9-tetrahydro-2,9-diazafluoren | 5,00 | 0,0026 | 0,013000 |
| 52 | 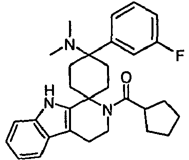 | 1,1-(3-Dimethylamino-3-(3-fluorophenyl)pentamethylen)-2-(cyclopentylcarbonyl)-1,3,4,9-tetrahydro-2,9-diazafluoren | 4,78 | 0,0067 | 0,032 |
| 53 | 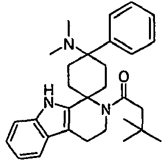 | 1,1-(3-Dimethylamino-3-phenylpentamethylen)-2-(3,3-dimethylbutanoyl)-1,3,4,9-tetrahydro-2,9-diazafluoren | 3,54 | 0,0048 | 0,017 |
| 54 | 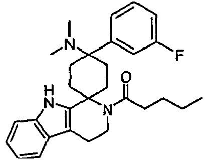 | 1,1-(3-Dimethylamino-3-(3-fluorophenyl)pentamethylen)-2-(pentanoyl)-1,3,4,9-tetrahydro-2,9-diazafluoren | 3,42 | 0,0038 | 0,013 |
| 55 | 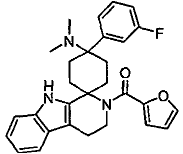 | 1,1-(3-Dimethylamino-3-(3-fluorophenyl)pentamethylen)-2-(2-furylcarbonyl)-1,3,4,9-tetrahydro-2,9-diazafluoren | 3,14 | 0,022 | 0,069 |
| 56 | 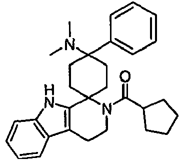 | 1,1-(3-Dimethylamino-3-phenylpentamethylen)-2-(2-furylcarbonyl)-1,3,4,9-tetrahydro-2,9-diazafluoren | 2,90 | 0,01 | 0,029 |
| 57 | 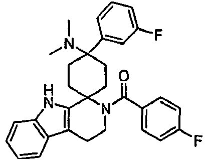 | 1,1-(3-Dimethylamino-3-(3-fluorophenyl)pentamethylen)-2-(4-fluorobenzoyl)-1,3,4,9-tetrahydro-2,9-diazafluoren | 2,83 | 0,0046 | 0,013 |
| 58 | 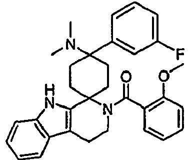 | 1,1-(3-Dimethylamino-3-(3-fluorophenyl)pentamethylen)-2-(2-methoxybenzoyl)-1,3,4,9-tetrahydro-2,9-diazafluoren | 2,78 | 0,00097 | 0,0027 |
| 59 | 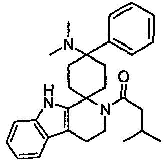 | 1,1-(3-Dimethylamino-3-phenylpentamethylen)-2-(3-methylbutanoyl)-1,3,4,9-tetrahydro-2,9-diazafluoren | 2,68 | 0,0041 | 0,011 |
| 60 | 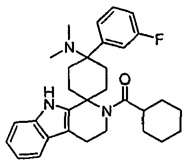 | 1,1-(3-Dimethylamino-3-(3-fluorophenyl)pentamethylen)-2-(cyclohexylcarbonyl)-1,3,4,9-tetrahydro-2,9-diazafluoren | 2,60 | 0,025 | 0,065 |
| 61 | 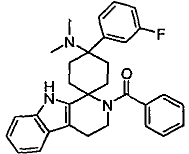 | 1,1-(3-Dimethylamino-3-(3-fluorophenyl)pentamethylen)-2-benzoyl-1,3,4,9-tetrahydro-2,9-diazafluoren | 2,50 | 0,0034 | 0,0085 |
| 62 | 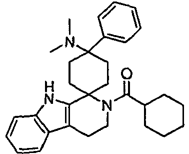 | 1,1-(3-Dimethylamino-3-phenylpentamethylen)-2-(cyclohexylcarbonyl)-1,3,4,9-tetrahydro-2,9-diazafluoren | 2,40 | 0,002 | 0,0048 |
| 63 | 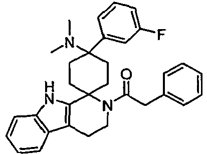 | 1,1-(3-Dimethylamino-3-(3-fluorophenyl)pentamethylen)-2-(phenylacetyl)-1,3,4,9-tetrahydro-2,9-diazafluoren | 2,35 | 0,002 | 0,0047 |
| 64 | 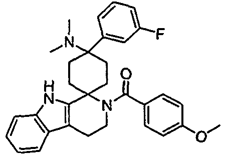 | 1,1-(3-Dimethylamino-3-(3-fluorophenyl)pentamethylen)-2-(4-methoxybenzoyl)-1,3,4,9-tetrahydro-2,9-diazafluoren | 2,29 | 0,038 | 0,087 |
| 65 | 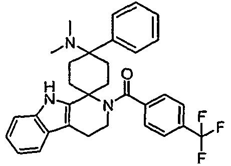 | 1,1-(3-Dimethylamino-3-phenylpentamethylen)-2-(4-trifluoromethylbenzoyl)-1,3,4,9-tetrahydro-2,9-diazafluoren | 2,18 | 0,0078 | 0,017 |
| 66 | 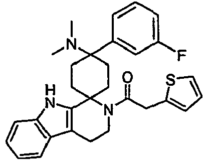 | 1,1-(3-Dimethylamino-3-(3-fluorophenyl)pentamethylen)-2-(thien-2-ylacetyl)-1,3,4,9-tetrahydro-2,9-diazafluoren | 2,12 | 0,017 | 0,036 |
| 67 | 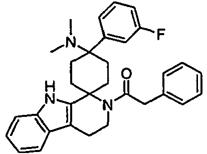 | 1,1-(3-Dimethylamino-3-(3-fluorophenyl)pentamethylen)-2-(phenylacetyl)-1,3,4,9-tetrahydro-2,9-diazafluoren | 2,07 | 0,0014 | 0,0029 |
| 68 | 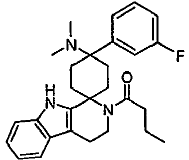 | 1,1-(3-Dimethylamino-3-(3-fluorophenyl)pentamethylen)-2-butanoyl-1,3,4,9-tetrahydro-2,9-diazafluoren | 2,00 | 0,0014 | 0,0028 |
| 69 | 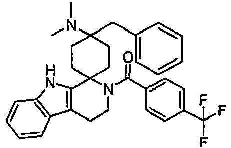 | 1,1-(3-Dimethylamino-3-benzylpentamethylen)-2-(4-trifluoromethylbenzoyl)-1,3,4,9-tetrahydro-2,9-diazafluoren | 1,91 | 0,011 | 0,021 |
| 70 | 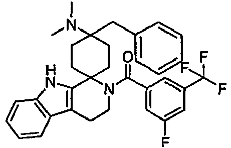 | 1,1-(3-Dimethylamino-3-benzyl-pentamethylen)-2-(3-fluoro-5-trifluoromethylbenzoyl)-1,3,4,9-tetrahydro-2,9-diazafluoren | 1,82 | 0,0077 | 0,014 |
| 71 | 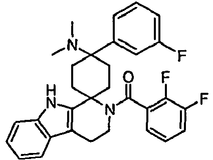 | 1,1-(3-Dimethylamino-3-(3-fluorophenyl)pentamethylen)-2-(2,3-difluorobenzoyl)-1,3,4,9-tetrahydro-2,9-diazafluoren | 1,79 | 0,0014 | 0,0025 |
| 72 | 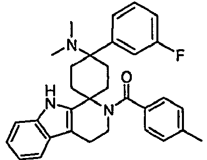 | 1,1-(3-Dimethylamino-3-(3-fluorophenyl)pentamethylen)-2-(4-methylbenzoyl)-1,3,4,9-tetrahydro-2,9-diazafluoren | 1,77 | 0,026 | 0,046 |
| 73* | 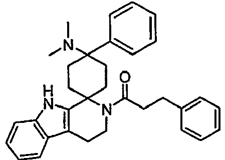 | 1,1-(3-Dimethylamino-3-phenylpentamethylen)-2-(3-phenylpropanoyl)-1,3,4,9-tetrahydro-2,9-diazafluoren | 1,64 | 0,028 | 0,046 |
| 74* | 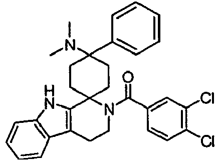 | 1,1-(3-Dimethylamino-3-phenylpentamethylen)-2-(3,4-dichlorobenzoyl)-1,3,4,9-tetrahydro-2,9-diazafluoren | 1,64 | 0,014 | 0,023 |
| 75* | 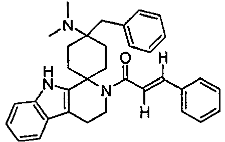 | 1,1-(3-Dimethylamino-3-benzylpentamethylen)-2-cinnamoyl-1,3,4,9-tetrahydro-2,9-diazafluoren | 1,54 | 0,024 | 0,037 |
| 76* | 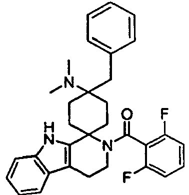 | 1,1-(3-Dimethylamino-3-benzylpentamethylen)-2-(2,6-difluorobenzoyl)-1,3,4,9-tetrahydro-2,9-diazafluoren | 1,54 | 0,054 | 0,083 |
| 77* | 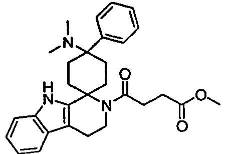 | 1,1-(3-Dimethylamino-3-phenylpentamethylen)-2-(3-methoxycarbonylpropanoyl)-1,3,4,9-tetrahydro-2,9-diazafluoren | 1,50 | 0,01 | 0,015 |
| 78* | 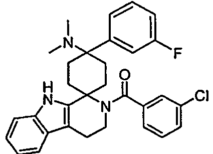 | 1,1-(3-Dimethylamino-3-(3-fluorophenyl)pentamethylen)-2-(3-chlorobenzoyl)-1,3,4,9-tetrahydro-2,9-diazafluoren | 1,50 | 0,018 | 0,027 |
| 79* | 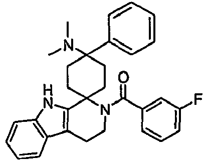 | 1,1-(3-Dimethylamino-3-phenylpentamethylen)-2-(3-fluorobenzoyl)-1,3,4,9-tetrahydro-2,9-diazafluoren | 1,50 | 0,0028 | 0,0042 |
| 80* | 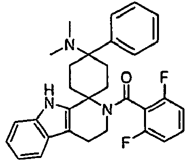 | 1,1-(3-Dimethylamino-3-phenylpentamethylen)-2-(2,6-difluorobenzoyl)-1,3,4,9-tetrahydro-2,9-diazafluoren | 1,50 | 0,01 | 0,015 |
| 81* | 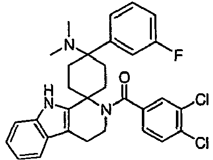 | 1,1-(3-Dimethylamino-3-(3-fluorophenyl)pentamethylen)-2-(3,4-dichlorobenzoyl)-1,3,4,9-tetrahydro-2,9-diazafluoren | 1,37 | 0,019 | 0,026 |
| 82* | 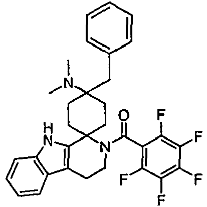 | 1,1-(3-Dimethylamino-3-benzylpentamethylen)-2-(2,3,4,5,6-pentafluorobenzoyl)-1,3,4,9-tetrahydro-2,9-diazafluoren | 1,35 | 0,06 | 0,081 |
| 83* | 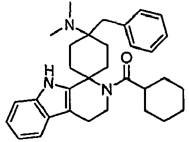 | 1, 1-(3-Dimethylamino-3-benrylpentamethylen)-2-cyclohexylcarbonyl-1,3,4,9-tetrahydro-2,9-diazafluoren | 1,31 | 0,013 | 0,017 |
| 84* | 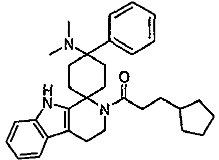 | 1,1-(3-Dimethylamino-3-phenylpentamethylen)-2-(3-cyclopentylpropanoyl)-1,3,4,9-tetrahydro-2,9-diazafluoren | 1,29 | 0,0093 | 0,012 |
| 85* | 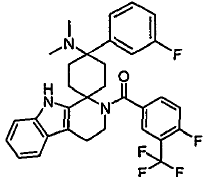 | 1,1-(3-Dimethylamino-3-(3-fluorophenyl)pentamethylen)-2-(4-fluoro-3-trifluoromethyl-benzoyl)-1,3,4,9-tetrahydro-2,9-diazafluoren | 1,28 | 0,0071 | 0,0091 |
| 86* | 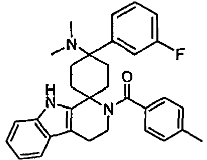 | 1,1-(3-Dimethylamino-3-(3-fluorophenyl)pentamethylen)-2-(4-methylbenzoyl)-1,3,4,9-tetrahydro-2,9-diazafluoren | 1,24 | 0,0055 | 0,0068 |
| 87* | 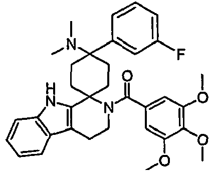 | 1,1-(3-Dimethylamino-3-(3-fluorophenyl)pentamethylen)-2-(3,4,5-trimethoxybenzoyl)-1,3,4,9-tetrahydro-2,9-diazafluoren | 1,21 | 0,019 | 0,023 |
| 88* | 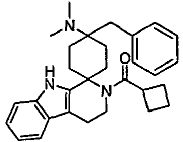 | 1,1-(3-Dimethylamino-3-benzylpentamethylen)-2-cyclobutylcarbonyl-1,3,4,9-tetrahydro-2,9-diazafluoren | 1,17 | 0,041 | 0,048 |
| 89* | 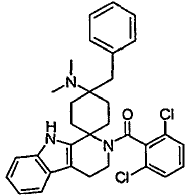 | 1,1-(3-Dimethylamino-3-benzylpentamethylen)-2-(2,6-dichlorobenzoyl)-1,3,4,9-tetrahydro-2,9-diazafluoren | 1,14 | 0,029 | 0,033 |
| 90* | 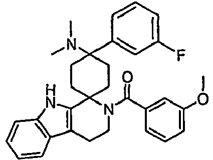 | 1,1-(3-Dimethylamino-3-(3-fluorophenyl)pentamethylen)-2-(3-methoxybenzoyl)-1,3,4,9-tetrahydro-2,9-diazafluoren | 1,12 | 0,098 | 0,11 |
| 91* | 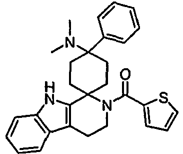 | 1,1-(3-Dimethylamino-3-phenylpentamethylen)-2-(2-thienylcarbonyl)-1,3,4,9-tetrahydro-2,9-diazafluoren | 1,09 | 0,032 | 0,035 |
| 92* | 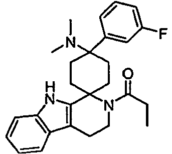 | 1,1-(3-Dimethylamino-3-(3-fluorophenyl)pentamethylen)-2-propanoyl-1,3,4,9-tetrahydro-2,9-diazafluoren | 1,07 | 0,057 | 0,061 |
| 93* | 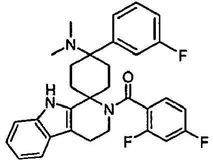 | 1,1-(3-Dimethylamino-3-(3-fluorophenyl)pentamethylen)-2-(2,4-difluorobenzoyl)-1,3,4,9-tetrahydro-2,9-diazafluoren | 1,06 | 0,016 | 0,017 |
| 94* | 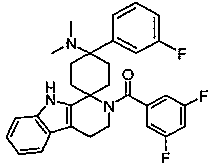 | 1,1-(3-Dimethylamino-3-(3-fluorophenyl)pentamethylen)-2-(3,5-difluorobenzoyl)-1,3,4,9-tetrahydro-2,9-diazafluoren | 1,06 | 0,0035 | 0,0037 |
| 95* | 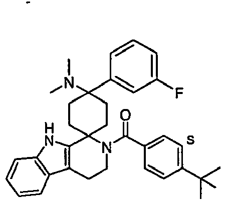 | 1,1-(3-Dimethylamino-3-(3-fluorophenyl)pentamethylen)-2-(4-tert.butylbenzoyl)-1,3,4,9-tetrahydro-2,9-diazafluoren | 1,06 | 0,0090 | 0,0095 |
| 96* | 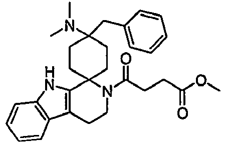 | 1,1-(3-Dimethylamino-3-benzylpentamethylen)-2-(3-methoxycarbonylpropanoyl)-1,3,4,9-tetrahydro-2,9-diazafluoren | 1,05 | 0,066 | 0,069 |
| 97* | 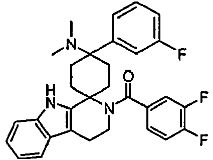 | 1,1-(3-Dimethylamino-3-(3-fluorophenyl)pentamethylen)-2-(3,4-difluorobenzoyl)-1,3,4,9-tetrahydro-2,9-diazafluoren | 1,04 | 0,0023 | 0,0024 |
| 98* | 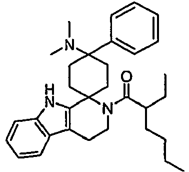 | 1,1-(3-Dimethylamino-3-phenylpentamethylen)-2-(2-ethylhexanoyl)-1,3,4,9-tetrahydro-2,9-diazafluoren | 1,04 | 0,047 | 0,049 |
| 99* | 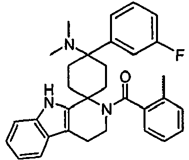 | 1,1-(3-Dimethylamino-3-(3-fluorophenyl)pentamethylen)-2-(2-methylbenzoyl)-1,3,4,9-tetrahydro-2,9-diazafluoren | 1,02 | 0,0092 | 0,0094 |
| 100* | 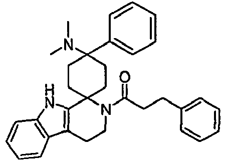 | 1,1-(3-Dimethylamino-3-phenylpentamethylen)-2-(3-phenylpropanoyl)-1,3,4,9-tetrahydro-2,9-diazafluoren | 1,00 | 0,04 | 0,04 |
| 101* | 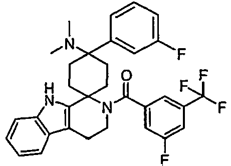 | 1,1-(3-Dimethylamino-3-(3-fluorophenyl)pentamethylen)-2-(3-fluoro-5-trifluoro-methylbenzoyl)-1,3,4,9-tetrahydro-2,9-diazafluoren | 1,00 | 0,0033 | 0,0033 |
| 102* | 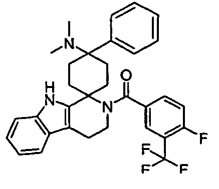 | 1,1-(3-Dimethylamino-3-phenylpentamethylen)-2-(3-fluoro-5-trifluoro-methylbenzoyl)-1,3,4,9-tetrahydro-2,9-diazafluoren | 1,00 | 0,036 | 0,036 |
| 103* | 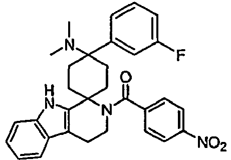 | 1,1-(3-Dimethylamino-3-(3-fluorophenyl)pentamethylen)-2-(4-nitrobenzoyl)-1,3,4,9-tetrahydro-2,9-diazafluoren | 0,97 | 0,0064 | 0,0062 |
| 104* | 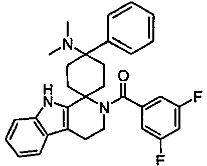 | 1,1-(3-Dimethylamino-3-phenylpentamethylen)-2-(3,5-difluorobenzoyl)-1,3,4,9-tetrahydro-2,9-diazafluoren | 0,95 | 0,002 | 0,0019 |
| 105* | 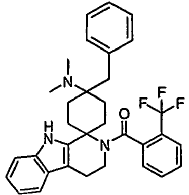 | 1,1-(3-Dimethylamino-3-benzylpentamethylen)-2-(2-trifluoromethylbenzoyl)-1,3,4,9-tetrahydro-2,9-diazafluoren | 0,95 | 0,019 | 0,018 |
| 106* | 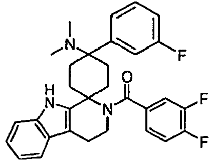 | 1,1-(3-Dimethylamino-3-(3-fluorophenyl)pentamethylen)-2-(3,4-difluorobenzoyl)-1,3,4,9-tetrahydro-2,9-diazafluoren | 0,94 | 0,0035 | 0,0033 |
| 107* | 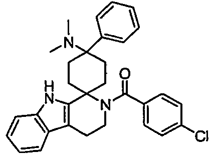 | 1,1-(3-Dimethylamino-3-phenylpentamethylen)-2-(4-chlorobenzoyl)-1,3,4,9-tetrahydro-2,9-diazafluoren | 0,91 | 0,011 | 0,010 |
| 108* | 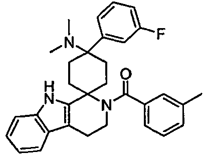 | 1,1-(3-Dimethylamino-3-(3-fluorophenyl)pentamethylen)-2-(3-methylbenzoyl)-1,3,4,9-tetrahydro-2,9-diazafluoren | 0,91 | 0,011 | 0,010 |
| 109* | 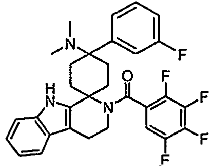 | 1,1-(3-Dimethylamino-3-(3-fluorophenyl)pentamethylen)-2-(2,3,4,5-tetrafluorobenzoyl)-1,3,4,9-tetrahydro-2,9-diazafluoren | 0,90 | 0,002 | 0,0018 |
| 110* | 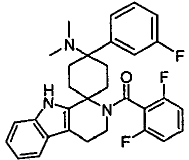 | 1,1-(3-Dimethylamino-3-(3-fluorophenyl)pentamethylen)-2-(2,6-difluorobenzoyl)-1,3,4,9-tetrahydro-2,9-diazafluoren | 0,88 | 0,0089 | 0,0078 |
| 111* | 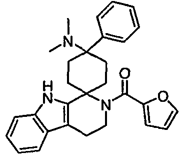 | 1,1-(3-Dimethylamino-3-phenylpentamethylen)-2-(2-furylcarbonyl)-1,3,4,9-tetrahydro-2,9-diazafluoren | 0,87 | 0,039 | 0,034 |
| 112* | 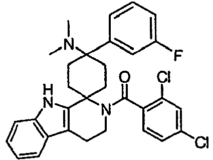 | 1,1-(3-Dimethylamino-3-(3-fluorophenyl)pentamethylen)-2-(2,4-dichlorobenzoyl)-1,3,4,9-tetrahydro-2,9-diazafluoren | 0,86 | 0,022 | 0,019 |
| 113* | 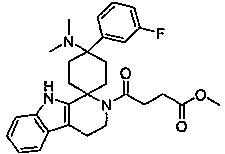 | 1,1-(3-Dimethylamino-3-(3-fluorophenyl)pentamethylen)-2-(3-methoxycarbonylpropanoyl)-1,3,4,9-tetrahydro-2,9-diazafluoren | 0,86 | 0,029 | 0,025 |
| 114* | 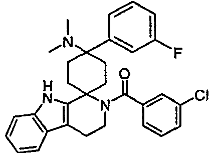 | 1,1-(3-Dimethylamino-3-(3-fluorophenyl)pentamethylen)-2-(3-chlorobenzoyl)-1,3,4,9-tetrahydro-2,9-diazafluoren | 0,83 | 0,0075 | 0,0062 |
| 115* | 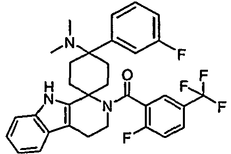 | 1,1-(3-Dimethylamino-3-(3-fluorophenyl)pentamethylen)-2-(2-fluoro-5-trifluoromethylbenzoyl)-1,3,4,9-tetrahydro-2,9-diazafluoren | 0,80 | 0,11 | 0,088 |
| 116* | 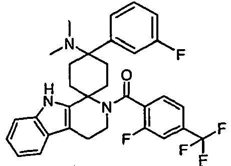 | 1,1-(3-Dimethylamino-3-(3-fluorophenyl)pentamethylen)-2-(2-fluoro-4-trifluoro-methylbenzoyl)-1,3,4,9-tetrahydro-2,9-diazafluoren | 0,80 | 0.0044 | 0,0035 |
| 117* | 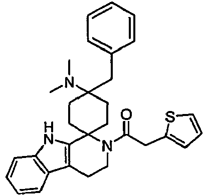 | 1,1-(3-Dimethylamino-3-benzylpentamethylen)-2-(2-thienylacetyl)-1,3,4,9-tetrahydro-2,9-diazafluoren | 0,79 | 0,014 | 0,011 |
| 118* | 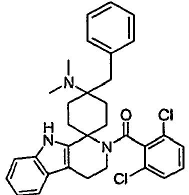 | 1,1-(3-Dimethylamino-3-benzylpentamethylen)-2-(2,6-dichlorobenzoyl)-1,3,4,9-tetrahydro-2,9-diazafluoren | 0,78 | 0,093 | 0,073 |
| 119* | 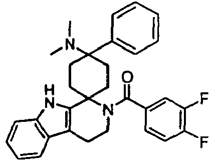 | 1,1-(3-Dimethylamino-3-phenylpentamethylen)-2-(3,4-difluorobenzoyl)-1,3,4,9-tetrahydro-2,9-diazafluoren | 0,76 | 0,0025 | 0,0019 |
| 120* | 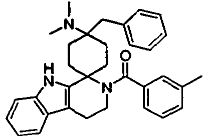 | 1,1-(3-Dimethylamino-3-benzylpentamethylen)-2-(3-methylbenzoyl)-1,3,4,9-tetrahydro-2,9-diazafluoren | 0,75 | 0,016 | 0,012 |
| 121* | 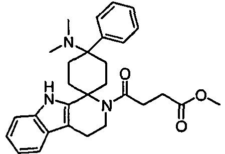 | 1,1-(3-Dimethylamino-3-phenylpentamethylen)-2-(3-methoxycarbonylpropanoyl)-1,3,4,9-tetrahydro-2,9-diazafluoren | 0,71 | 0,021 | 0,015 |
| 122* | 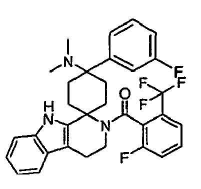 | 1,1-(3-Dimethylamino-3-(3-fluorophenyl)pentamethylen)-2-(2-fluoro-6-trifluoromethylbenzoyl)-1,3,4,9-tetrahydro-2,9-diazafluoren | 0,67 | 0,0054 | 0,0036 |
| 123* | 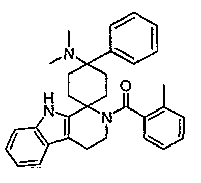 | 1,1-(3-Dimethylamino-3-phenylpentamethylen)-2-(2-methylbenzoyl)-1,3,4,9-tetrahydro-2,9-diazafluoren | 0,65 | 0,031 | 0,020 |
| 124* | 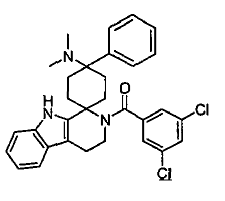 | 1,1-(3-Dimethylamino-3-phenylpentamethylen)-2-(3,5-dichlorobenzoyl)-1,3,4,9-tetrahydro-2,9-diazafluoren | 0.64 | 0,0550 | 0,035 |
| 125* | 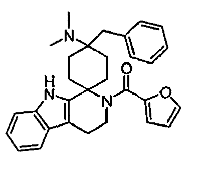 | 1,1-(3-Dimethylamino-3-benzylpentamethylen)-2-(2-furylcarbonyl)-1,3,4,9-tetrahydro-2,9-diazafluoren | 0,63 | 0,0054 | 0,0034 |
| 126* | 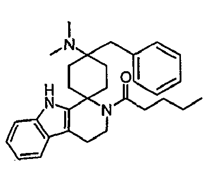 | 1,1-(3-Dimethylamino-3-benzylpentamethylen)-2-(pentanoyl)-1,3,4,9-tetrahydro-2,9-diazafluoren | 0,63 | 0,0043 | 0,0027 |
| 127* | 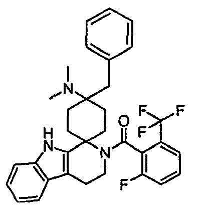 | 1,1-(3-Dimethylamino-3-benzylpentamethylen)-2-(6-fluoro-2-trifluoromethylbenzoyl)-1,3,4,9-tetrahydro-2,9-diazafluoren | 0,62 | 0,055 | 0,034 |
| 128* | 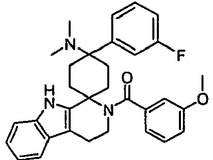 | 1,1-(3-Dimethylamino-3-(3-fluorophenyl)pentamethylen)-2-(3-methoxybenzoyl)-1,3,4,9-tetrahydro-2,9-diazafluoren | 0,57 | 0,028 | 0,016 |
| 129* | 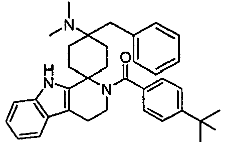 | 1,1-(3-Dimethylamino-3-benzylpentamethylen)-2-(4-tert.butylbenzoyl)-1,3,4,9-tetrahydro-2,9-diazafluoren | 0,54 | 0,11 | 0,059 |
| 130* | 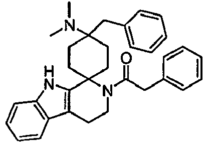 | 1,1-(3-Dimethylamino-3-benzylpentamethylen)-2-(phenylacetyl)-1,3,4,9-tetrahydro-2,9-diazafluoren | 0,54 | 0,0099 | 0,0053 |
| 131* | 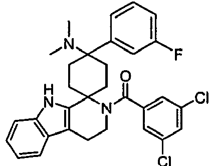 | 1,1-(3-Dimethylamino-3-(3-fluorophenyl)pentamethylen)-2-(3,5-dichlorobenzoyl)-1,3,4,9-tetrahydro-2,9-diazafluoren | 0,53 | 0,030 | 0,016 |
| 132* | 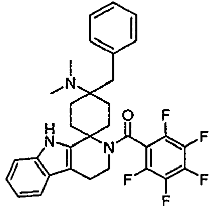 | 1,1-(3-Dimethylamino-3-benzylpentamethylen)-2-(2,3,4,5,6-pentafluorobenzoyl)-1,3,4,9-tetrahydro-2,9-diazafluoren | 0,52 | 0,075 | 0,039 |
| 133* | 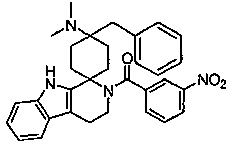 | 1,1-(3-Dimethylamino-3-benzylpentamethylen)-2-(3-nitrobenzoyl)-,3,4,9-tetrahydro-2,9-diazafluoren | 0,52 | 0,017 | 0,0088 |
| 134* | 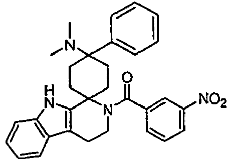 | 1,1-(3-Dimethylamino-3-phenylpentamethylen)-2-(3-nitrobenzoyl)-1,3,4,9-tetrahydro-2,9-diazafluoren | 0,51 | 0,069 | 0,035 |
| 135* | 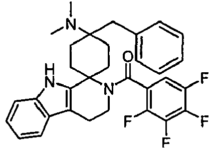 | 1,1-(3-Dimethylamino-3-benzylpentamethylen)-2-(2,3,4,5-tetrafluorobenzoyl)-1,3,4,9-tetrahydro-2,9-diazafluoren | 0,50 | 0,030 | 0,015 |
| 136* | 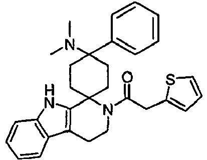 | 1,1-(3-Dimethylamino-3-phenylpentamethylen)-2-(2-thienylacetyl)-1,3,4,9-tetrahydro-2,9-diazafluoren | 0,48 | 0,0069 | 0,0033 |
| 137* | 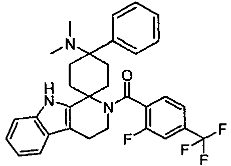 | 1, 1-(3-Dimethylamino-3-phenylpentamethylen)-2-(2-fluoro-4-trifluoromethylbenzoyl)-1,3,4,9-tetrahydro-2,9-diazafluoren | 0,48 | 0,065 | 0,031 |
| 138* | 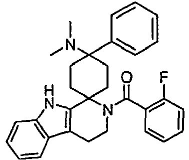 | 1,1-(3-Dimethylamino-3-phenylpentamethylen)-2-(2-fluorobenzoyl)-1,3,4,9-tetrahydro-2,9-diazafluoren | 0,47 | 0,018 | 0,0084 |
| 139* | 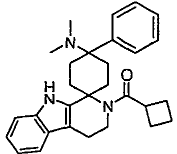 | 1,1-(3-Dimethylamino-3-phenylpentamethylen)-2-(cyclobutylcarbonyl)-1,3,4,9-tetrahydro-2,9-diazafluoren | 0,45 | 0,075 | 0,034 |
| 140* | 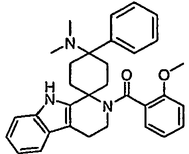 | 1,1-(3-Dimethylamino-3-phenylpentamethylen)-2-(2-methoxybenzoyl)-1,3,4,9-tetrahydro-2,9-diazafluoren | 0,43 | 0,046 | 0,020 |
| 141* | 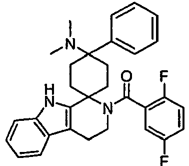 | 1,1-(3-Dimethylamino-3-phenylpentamethylen)-2-(2,5-difluorobenzoyl)-1,3,4,9-tetrahydro-2,9-diazafluoren | 0,42 | 0,0045 | 0,0019 |
| 142* | 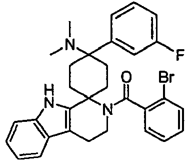 | 1,1-(3-Dimethylamino-3-(3-fluorophenyl)pentamethylen)-2-(2-bromobenzoyl)-1,3,4,9-tetrahydro-2,9-diazafluoren | 0,41 | 0,046 | 0,019 |
| 143* | 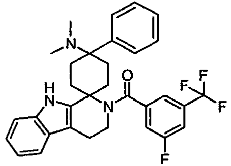 | 1,1-(3-Dimethylamino-3-phenylpentamethylen)-2-(3-fluoro-5-trifluoromethylbenzoyl)-1,3,4,9-tetrahydro-2,9-diazafluoren | 0,41 | 0,037 | 0,015 |
| 144* | 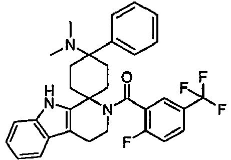 | 1,1-(3-Dimethylamino-3-phenylpentamethylen)-2-(2-fluoro-5-trifluoromethylbenzoyl)-1,3,4,9-tetrahydro-2,9-diazafluoren | 0,39 | 0,049 | 0,019 |
| 145* | 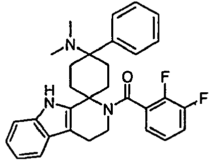 | 1,1-(3-Dimethylamino-3-phenylpentamethylen)-2-(2,3-difluorobenzoyl)-1,3,4,9-tetrahydro-2,9-diazafluoren | 0,33 | 0,029 | 0,0096 |

### Jumping-test an der Maus: Versuch zur Bestimmung der körperlichen Abhängigkeit (Saelens JK, Arch Int Pharmacodyn 190: 213-218, 1971)

Die Testsubstanzen werden insgesamt 7x über zwei Tage intraperitoneal appliziert. 5 Applikationen erfolgen am ersten Tag um 9:00, 10:00, 11:00, 13:00 und 15:00 Uhr und am zweiten Tag um 9:00 und 11:00 Uhr. Die ersten 3 Applikationen werden in aufsteigenden Dosierungen (Dosierungsschema) gegeben und dann weiter in der Dosierung der dritten. Der Entzug wird 2 Stunden nach der letzten Substanzaplikation mit Naloxon 30 mg/kg (i.p.) präzipititert. Unmittelbar danach werden die Tiere einzeln in durchsichtige Beobachtungsboxen (Höhe 40 cm, Durchmesser 15 cm) gesetzt und die Sprungreaktionen über 15 Minuten in jeweils 5-Minuten- Perioden gezählt. Morphin wird in einer Dosierung als Vergleich/Standard mitgeführt.

Die Quantifizierung des Entzugs erfolgt über die Anzahl der Sprünge 0 bis 10 min. nach Naloxonapplikation. Die Anzahl der Tiere pro Gruppe mit mehr als 10 Sprüngen/ 10 min wird bestimmt und als "% positive Tiere" dokumentiert. Außerdem wird die durchschnittliche Sprung-Frequenz in der Gruppe errechnet. Pro Gruppe werden 12 Tiere eingesetzt.

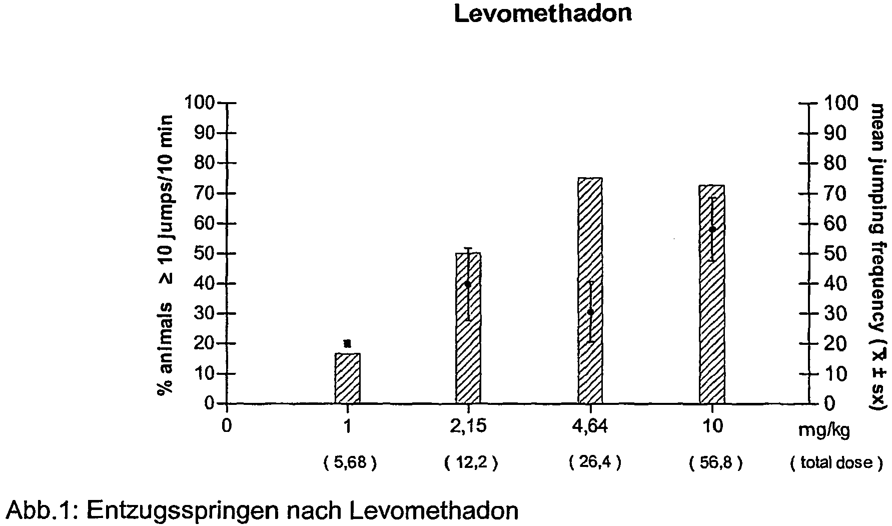

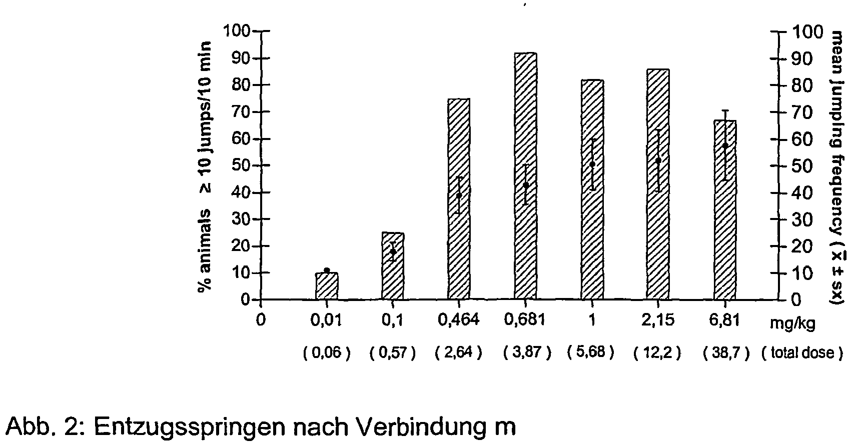

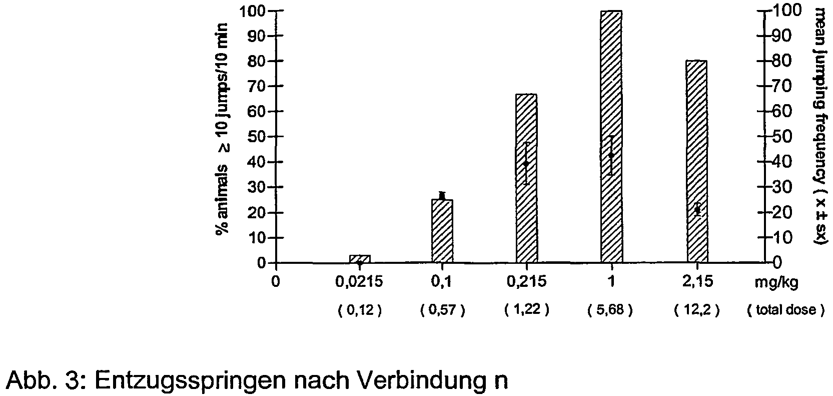

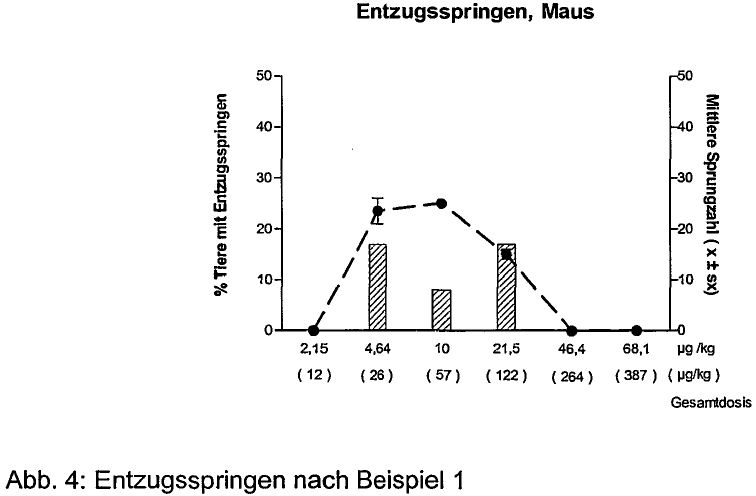

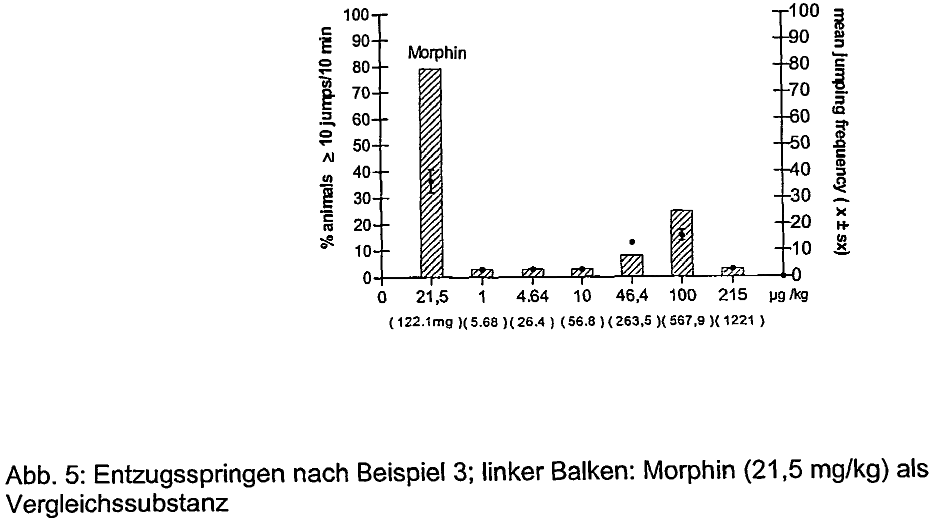

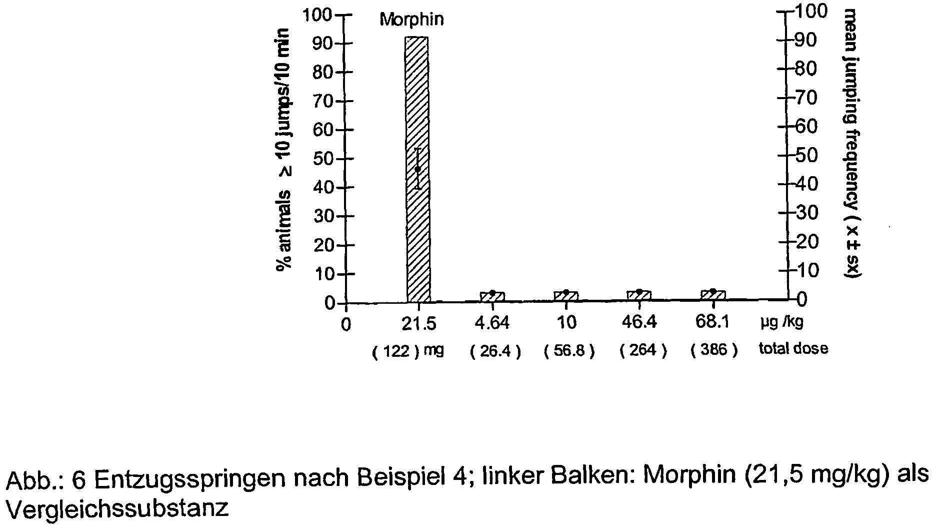

### Untersuchungen zu cardiovaskulären Effekten

### Methodenbeschreibung EKG, wacher Hund:

Substanzeffekte auf Herzfrequenz und elektrokardiographische Parameter wurden am wachen Beagle-Hund untersucht.

Die Messung des Elektrokardiogramms (EKG) erfolgte als bipolare Extremitätenableitung nach Einthoven (Ableitung II). Dazu wurden die Beagle-Hunde in einer speziellen hängemattenartigen Halterung (Animal sling, Havard Instruments, ZAK, Marktheidenfeld, Deutschland) gesetzt und leicht fixiert. An der linken und rechten Vorderextremität wurde jeweils eine Messelektrode, sowie an der linken Hinterextremität eine Referenzelektrode angebracht. Alle Elektroden waren Plattenelektrode, die auf der rasierten Haut mittels Gummimanschetten fixiert wurden. Die Plattenelektroden wurden an einen EKG-Vorverstärker angeschlossen und die Signale kontinuierlich mit Hilfe eines computergestützten Datenaufnahme und -archivierungssystems (PO-NE-MAH, Gould-Instrument Systems, USA) digitalisiert (Digitalisierungsfrequenz: 2 kHz). Pro Messzeitpunkt wurden aus 10 aufeinander folgenden EKG-Intervallen die Mittelwerte folgender Parameter bestimmt: RR-Intervall, PR-Intervall, QRS-Intervall und QT-Intervall. Die Frequenzkorrektur des QT-Intervalls erfolgte nach der Korrekturformel von Van de Water et al. (1989).

Die Testsubstanzen wurden jeweils als i.v. Kurzzeitinfusion (15 min) über die V. cephalica antibrachii appliziert. Zur Quantifizierung von Substanzeffekten wurden über einen Zeitraum von 60 min nach Substanzapplikation die Änderungen der EKG-Parameter relativ zum Basiswert vor Substanzapplikation registriert. Die statistische Auswertung erfolgte mittels Varianzanalyse (ANOVA) im Vergleich zu zeitgleich verlaufenden Vehikelkontrollmessungen. (Van de Water A, Verheyen J, Xhonneux R, Reneman RS. An improved method to correct the QT interval of the electrocardiogram for changes in heart rate. J Pharmacol Methods. 1989 Nov;22(3):207-17).

### Patch-clamp, hERG-Strom

Methodenbeschreibung in: Bischoff U, Schmidt C, Netzer R, Pongs O. Effects of fluoroquinolones on HERG currents. Eur J Pharmacol. 2000;406(3):341-3.

### Blutdruck, wache Ratte

Methodenbeschreibung in: Weeks JR, Compton LD. The cardiovascular pharmacology of prostacyclin (PG12) in the rat. Prostaglandins 1979;17(4):501-13.

**Tab. A: Zusammenfassung der cardiovasculären Effekte von Beispiel 1:**

| **Modell** | **Dosis** | **Beobachtung** | |
|---|---|---|---|
| Patch clamp, hERG-Strom | | IC50 >> 10 µM (10 µM: 29±7% Hemmung) | |
| Blutdruck, Herzfrequenz, wache Ratte | bis 14.7 µg/kg i.v. ** | Kein Effekt | |
| EKG, wacher Hund | 10 µg/kg i.v. | ● | Sign. Bradykardie (Herzfrequenz: -40%) |
| | | ● | Nichtsignifikante Verlängerung der QTc [van der Water] |
| | | ● | Leichte (<10%), aber signifikante Erhöhung der QRS-Zeit |
| | | ● | Kein Effekt auf das PR-Intervall |

| | | | |
|---|---|---|---|
| ** Tail-flick, rat: EC₅₀ = 1.44 µg/kg i.v. | | | |

Zum Vergleich: Der IC₅₀-Wert für LAAM liegt bei 2 µM (Wechselwirkungen von LAAM/HERG-Kanal s. Jiesheng et al., Eur. J. Pharmacol. 2003, 458, 25-29), der IC50-Wert von Methadon bei 9,8 µM (s. Katchman et al., J. Pharmacol. Exp. Ther. 2002, 303, 688-694).

An der wachen Ratte werden bei einer Gabe von bis zu einer Größenordnung höher als der EC₅₀-Wert im Tail-flick an der Ratte von Beispiel 1 keine cardiovaskulären Effekte beobachtet.

## Patentansprüche

1. Spirocyclisches Cyclohexan-Derivat ausgewählt aus der Gruppe bestehend aus
1,1-(3-Dimethylamino-3-(3-fluorophenyl)pentamethylen)-2-cinnamoyl-1,3,4,9-tetrahydro-2,9-diazafluoren;
1,1-(3-Dimethylamino-3-(3-fluorophenyl)pentamethylen)-2-(3,3-dimethylbutanoyl)-1,3,4,9-tetrahydro-2,9-diazafluoren;
1,1-(3-Dimethylamino-3-(3-fluorophenyl)pentamethylen)-2-(cyclopentylcarbonyl)-1,3,4,9-tetrahydro-2,9-diazafluoren;
1,1-(3-Dimethylamino-3-phenylpentamethylen)-2-(3,3-dimethylbutanoyl)-1,3,4,9-tetrahydro-2,9-diazafluoren;
1,1-(3-Dimethylamino-3-(3-fluorophenyl)pentamethylen)-2-(pentanoyl)-1,3,4,9-tetrahydro-2,9-diazafluoren;
1,1-(3-Dimethylamino-3-(3-fluorophenyl)pentamethylen)-2-(2-furylcarbonyl)-1,3,4,9-tetrahydro-2,9-diazafluoren;
1,1-(3-Dimethylamino-3-phenylpentamethylen)-2-(2-furylcarbonyl)-1,3,4,9-tetrahydro-2,9-diazafluoren;
1,1-(3-Dimethylamino-3-(3-fluorophenyl)pentamethylen)-2-(4-fluorobenzoyl)-1,3,4,9-tetrahydro-2,9-diazafluoren;
1,1-(3-Dimethylamino-3-(3-fluorophenyl)pentamethylen)-2-(2-methoxybenzoyl)-1,3,4,9-tetrahydro-2,9-diazafluoren;
1,1-(3-Dimethylamino-3-phenylpentamethylen)-2-(3-methylbutanoyl)-1,3,4,9-tetrahydro-2,9-diazafluoren;
1,1-(3-Dimethylamino-3-(3-fluorophenyl)pentamethylen)-2-(cyclohexylcarbonyl)-1,3,4,9-tetrahydro-2,9-diazafluoren;
1,1-(3-Dimethylamino-3-(3-fluorophenyl)pentamethylen)-2-benzoyl-1,3,4,9-tetrahydro-2,9-diazafluoren;
1,1-(3-Dimethylamino-3-phenylpentamethylen)-2-(cyclohexylcarbonyl)-1,3,4,9-tetrahydro-2,9-diazafluoren;
1,1-(3-Dimethylamino-3-(3-fluorophenyl)pentamethylen)-2-(phenylacetyl)-1,3,4,9-tetrahydro-2,9-diazafluoren;
1.1-(3,Dimethylamino-3-(3-fluorophenyl)pentamethylen)-2-(4-methoxybenzoyl)-1,3,4,9-tetrahydro-2,9-diazafluoren;
1,1-(3-Dimethylamino-3-phenylpentamethylen)-2-(4-trifluoromethylbenzoyl)-1,3,4,9-tetrahydro-2,9-diazafluoren;
1,1-(3-Dimethylamino-3-(3-fluorophenyl)pentamethylen)-2-(thien-2-ylacetyl)-1,3,4,9-tetrahydro-2,9-diazafluoren;
1,1-(3-Dimethylamino-3-(3-fluorophenyl)pentamethylen)-2-(phenylacetyl)-1,3,4,9-tetrahydro-2,9-diazafluoren;
1,1-(3-Dimethylamino-3-(3-fluorophenyl)pentamethylen)-2-butanoyl-1,3,4,9-tetrahydro-2,9-diazafluoren;
1,1-(3-Dimethylamino-3-benzylpentamethylen)-2-(4-trifluoromethylbenzoyl)-1,3,4,9-tetrahydro-2,9-diazafluoren;
1,1-(3-Dimethylamino-3-benzylpentamethylen)-2-(3-fluoro-5-trifluoromethylbenzoyl)-1,3,4,9-tetrahydro-2,9-diazafluoren;
1,1-(3-Dimethylamino-3-(3-fluorophenyl)pentamethylen)-2-(2,3-difluorobenzoyl)-1,3,4,9-tetrahydro-2,9-diazafluoren;
1,1-(3-Dimethylamino-3-(3-fluorophenyl)pentamethylen)-2-(4-methylbenzoyl)-1,3,4,9-tetrahydro-2,9-diazafluoren;
in Form der reinen Diastereomere, des Racemats, der reinen Enantiomere, oder in Form von Mischungen der Stereoisomere in einem beliebigen Mischungsverhältnis; als Basen oder in Form der Salze, insbesondere der physiologisch verträglichen Salze oder Salze physiologisch verträglicher Säuren oder Kationen; oder in Form der Solvate.

2. Spirocyclisches Cyclohexan-Derivat nach Anspruch 1, zur Anwendung bei der Behandlung von Substanzabhängigkeit.

3. Spirocyclisches Cyclohexan-Derivat zur Anwendung nach Anspruch 2, wobei es sich bei der Substanzabhängigkeit um Opiatabhängigkeit handelt.

4. Spirocyclisches Cyclohexan-Derivat zur Anwendung nach Anspruch 2 oder 3, wobei es sich bei der Substanzabhängigkeit um Abhängigkeit von Heroin, Morphin oder Methadon handelt.

## Claims

1. Spirocyclic cyclohexane derivative selected from the group consisting of
1,1-(3-dimethylamino-3-(3-fluorophenyl)pentamethylene)-2-cinnamoyl-1,3,4,9-tetrahydro-2,9-diazafluorene;
1,1-(3-dimethylamino-3-(3-fluorophenyl)pentamethylene)-2-(3,3-dimethylbutanoyl)-1,3,4,9-tetrahydro-2,9-diazafluorene;
1,1-(3-dimethylamino-3-(3-fluorophenyl)pentamethylene)-2-(cyclopentylcarbonyl)-1,3,4,9-tetrahydro-2,9-diazafluorene;
1,1-(3-dimethylamino-3-phenylpentamethylene)-2-(3,3-dimethylbutanoyl)-1,3,4,9-tetrahydro-2,9-diazafluorene;
1,1-(3-dimethylamino-3-(3-fluorophenyl)pentamethylene)-2-(pentanoyl)-1,3,4,9-tetrahydro-2,9-diazafluorene;
1,1-(3-dimethylamino-3-(3-fluorophenyl)pentamethylene)-2-(2-furylcarbonyl)-1,3,4,9-tetrahydro-2,9-diazafluorene;
1,1-(3-dimethylamino-3-phenylpentamethylene)-2-(2-furylcarbonyl)-1,3,4,9-tetrahydro-2,9-diazafluorene;
1,1-(3-dimethylamino-3-(3-fluorophenyl)pentamethylene)-2-(4-fluorobenzoyl)-1,3,4,9-tetrahydro-2,9-diazafluorene;
1,1-(3-dimethylamino-3-(3-fluorophenyl)pentamethylene)-2-(2-methoxybenzoyl)-1,3,4,9-tetrahydro-2,9-diazafluorene;
1,1-(3-dimethylamino-3-phenylpentamethylene)-2-(3-methylbutanoyl)-1,3,4,9-tetrahydro-2,9-diazafluorene;
1,1-(3-dimethylamino-3-(3-fluorophenyl)pentamethylene)-2-(cyclohexylcarbonyl)-1,3,4,9-tetrahydro-2,9-diazafluorene;
1,1-(3-dimethylamino-3-(3-fluorophenyl)pentamethylene)-2-benzoyl-1,3,4,9-tetrahydro-2,9-diazafluorene;
1,1-(3-dimethylamino-3-phenylpentamethylene)-2-(cyclohexylcarbonyl)-1,3,4,9-tetrahydro-2,9-diazafluorene;
1,1-(3-dimethylamino-3-(3-fluorophenyl)pentamethylene)-2-(phenylacetyl)-1,3,4,9-tetrahydro-2,9-diazafluorene;
1,1-(3-dimethylamino-3-(3-fluorophenyl)pentamethylene)-2-(4-methoxybenzoyl)-1,3,4,9-tetrahydro-2,9-diazafluorene;
1,1-(3-dimethylamino-3-phenylpentamethylene)-2-(4-trifluoromethylbenzoyl)-1,3,4,9-tetrahydro-2,9-diazafluorene;
1,1-(3-dimethylamino-3-(3-fluorophenyl)pentamethylene)-2-(thien-2-ylacetyl)-1,3,4,9-tetrahydro-2,9-diazafluorene;
1,1-(3-dimethylamino-3-(3-fluorophenyl)pentamethylene)-2-(phenylacetyl)-1,3,4,9-tetrahydro-2,9-diazafluorene;
1,1-(3-dimethylamino-3-(3-fluorophenyl)pentamethylene)-2-butanoyl-1,3,4,9-tetrahydro-2,9-diazafluorene;
1,1-(3-dimethylamino-3-benzylpentamethylene)-2-(4-trifluoromethylbenzoyl)-1,3,4,9-tetrahydro-2,9-diazafluorene;
1,1-(3-dimethylamino-3-benzylpentamethylene)-2-(3-fluoro-5-trifluoromethylbenzoyl)-1,3,4,9-tetrahydro-2,9-diazafluorene;
1,1-(3-dimethylamino-3-(3-fluorophenyl)pentamethylene)-2-(2,3-difluorobenzoyl)-1,3,4,9-tetrahydro-2,9-diazafluorene;
1,1-(3-dimethylamino-3-(3-fluorophenyl)pentamethylene)-2-(4-methylbenzoyl)-1,3,4,9-tetrahydro-2,9-diazafluorene;
in the form of the pure diastereomers, the racemate, the pure enantiomers, or in the form of mixtures of the stereoisomers in an arbitrary mixture ratio; as bases or in the form of the salts, in particular of the physiologically compatible salts or salts of physiologically compatible acids or cations; or in the form of the solvates.

2. Spirocyclic cyclohexane derivative according to claim 1 for use in the treatment of substance dependency.

3. Spirocyclic cyclohexane derivative for use according to claim 2, wherein the substance dependency is opiate dependency.

4. Spirocyclic cyclohexane derivative for use according to claim 2 or 3, wherein the substance dependency is heroin, morphine or methadone dependency.

## Revendications

1. Dérivé spirocyclique de cyclohexane choisi dans le groupe consistant en :
le 1,1-(3-diméthylamino-3-(3-fluorophényl)pentaméthylène)-2-cinnamoyl-1,3,4,9-tétrahydro-2,9-diazafluorène ;
le 1,1-(3-diméthylamino-3-(3-fluorophényl)pentaméthylène)-2-(3,3-diméthylbutanoyl)-1,3,4,9-tétrahydro-2,9-diazafluorène ;
le 1,1-(3-diméthylamino-3-(3-fluorophényl)pentaméthylène)-2-(cyclopentylcarbonyl)-1,3,4,9-tétrahydro-2,9-diazafluorène ;
le 1,1-(3-diméthylamino-3-phénylpentaméthylène)-2- (3,3-diméthylbutanoyl)-1,3,4,9-tétrahydro-2,9-diazafluorène ;
le 1,1-(3-diméthylamino-3-(3-fluorophényl)pentaméthylène)-2-(pentanoyl)-1,3,4,9-tétrahydro-2,9-diazafluorène ;
le 1,1-(3-diméthylamino-3-(3-fluorophényl)pentaméthylène)-2-(2-furylcarbonyl)-1,3,4,9-tétrahydro-2,9-diazafluorène ;
le 1,1-(3-diméthylamino-3-phénylpentaméthylène)-2-(2-furylcarbonyl)-1,3,4,9-tétrahydro-2,9-diazafluorène ;
le 1,1-(3-diméthylamino-3-(3-fluorophényl)pentaméthylène)-2-(4-fluorobenzoyl)-1,3,4,9-tétrahydro-2,9-diazafluorène ;
le 1,1-(3-diméthylamino-3-(3-fluorophényl)pentaméthylène)-2-(2-méthoxybenzoyl)-1,3,4,9-tétrahydro-2,9-diazafluorène ;
le 1,1-(3-diméthylamino-3-phénylpentaméthylène)-2-(3-méthylbutanoyl)-1,3,4,9-tétrahydro-2,9-diazafluorène ;
le 1,1-(3-diméthylamino-3-(3-fluorophényl)pentaméthylène)-2-(2-cyclohexylcarbonyl)-1,3,4,9-tétrahydro-2,9-diazafluorène ;
le 1,1-(3-diméthylamino-3-(3-fluorophényl)pentaméthylène)-2-benzoyl-1,3,4,9-tétrahydro-2,9-diazafluorène ;
le 1,1-(3-diméthylamino-3-phénylpentaméthylène)-2-(cyclohexylcarbonyl)-1,3,4,9-tétrahydro-2,9-diazafluorène ;
le 1,1-(3-diméthylamino-3-(3-fluorophényl)pentaméthylène)-2-(phénylacétyl)-1,3,4,9-tétrahydro-2,9-diazafluorène ;
le 1,1-(3-diméthylamino-3-(3-fluorophényl)pentaméthylène)-2-(4-méthoxybenzoyl)-1,3,4,9-tétrahydro-2,9-diazafluorène ;
le 1,1-(3-diméthylamino-3-phénylpentaméthylène)-2-(4-trifluorométhylbenzoyl)-1,3,4,9-tétrahydro-2,9-diazafluorène ;
le 1,1-(3-diméthylamino-3-(3-fluorophényl)pentaméthylène)-2-(thién-2-ylacétyl)-1,3,4,9-tétrahydro-2,9-diazafluorène ;
le 1,1-(3-diméthylamino-3-(3-fluorophényl)pentaméthylène)-2-(phénylacétyl)-1,3,4,9-tétrahydro-2,9-diazafluorène ;
le 1,1-(3-diméthylamino-3-(3-fluorophényl)pentaméthylène)-2-butanoyl-1,3,4,9-tétrahydro-2,9-diazafluorène ;
le 1,1-(3-diméthylamino-3-benzylpentaméthylène)-2-(4-trifluorométhylbenzoyl)-1,3,4,9-tétrahydro-2,9-diazafluorène ;
le 1,1-(3-diméthylamino-3-benzylpentaméthylène)-2-(3-fluoro-5-trifluorométhylbenzoyl)-1,3,4,9-tétrahydro-2,9-diazafluorène ;
le 1,1-(3-diméthylamino-3-(3-fluorophényl)pentaméthylène)-2-(2,3-difluorobenzoyl)-1,3,4,9-tétrahydro-2,9-diazafluorène ;
le 1,1-(3-diméthylamino-3-(3-fluorophényl)pentaméthylène)-2-(4-méthylbenzoyl)-1,3,4,9-tétrahydro-2,9-diazafluorène ;
sous la forme des diastéréoisomères purs, du racémate, des énantiomères purs, ou sous la forme de mélanges des stéréoisomères dans un quelconque rapport de mélange ; en tant que bases ou sous la forme des sels, en particulier des sels physiologiquement acceptables ou des sels d'acides ou cations physiologiquement acceptables ; ou sous la forme des produits de solvatation.

2. Dérivé spirocyclique de cyclohexane selon la revendication 1, destiné à être utilisé dans le traitement de la dépendance à des substances.

3. Dérivé spirocyclique de cyclohexane destiné à être utilisé selon la revendication 2, dans lequel la dépendance à des substances est la dépendance aux opiacés.

4. Dérivé spirocyclique de cyclohexane destiné à être utilisé selon la revendication 2 ou 3, dans lequel la dépendance à des substances est la dépendance à l'héroïne, la morphine ou la méthadone.
